# EUROPEAN PATENT SPECIFICATION

(11) **EP 4 196 016 B1**
(45) Date of publication and mention of the grant of the patent: **26.03.2025**
(21) Application number: 22724895.2
(22) Date of filing: 06.05.2022
(51) Int. Cl.: A61B 17/064, A61B 17/072, A61L 31/10, A61L 31/02, A61B 50/33, A61B 17/068, A61B 17/00, A61B 90/92, A61B 90/94, A61B 90/98, A61B 90/00, A61B 50/30, A61B 50/00

(54) **PACKAGING ASSEMBLIES FOR SURGICAL STAPLE CARTRIDGES CONTAINING BIOABSORBABLE STAPLES**
VERPACKUNGSANORDNUNGEN FÜR CHIRURGISCHE KLAMMERMAGAZINE MIT BIOABSORBIERBAREN KLAMMERN
ENSEMBLES D'EMBALLAGE POUR CARTOUCHES D'AGRAFES CHIRURGICALES CONTENANT DES AGRAFES BIOABSORBABLES

(30) Priority: 10.05.2021 US 202163186519 P; 12.04.2022 US 202217718884
(43) Date of publication of application: 21.06.2023
(73) Proprietor: Cilag GmbH International, 6300 Zug (CH)
(72) Inventor: RECTOR, Jason M., Cincinnati, Ohio 45242 (US); MCGHEE, Ryan W., Cincinnati, Ohio 45242 (US); ROLL, Benjamin A., Cincinnati, Ohio 45242 (US); MOSER, Sean J., Cincinnati, Ohio 45242 (US); SHELTON IV, Frederick E., Cincinnati, Ohio 45242 (US); HARRIS, Jason L., Cincinnati, Ohio 45242 (US); JONES, Shannon L., Cincinnati, Ohio 45242 (US); PATHAK, Shashi S., Cincinnati, Ohio 45242 (US)
(74) Representative: Carpmaels & Ransford LLP
(86) International application number: PCT/IB2022/054212
(87) International publication number: WO 2022/238841

(56) References cited:
- EP-A1- 3 072 459
- EP-A1- 3 756 613
- EP-A2- 3 135 208
- US-A- 4 511 035
- US-A- 4 730 726
- US-A- 5 322 161
- US-A1- 2012 241 491
- US-A1- 2020 205 825
- US-A1- 2020 261 080
- US-A1- 2020 405 301

## Description

### BACKGROUND

During a surgical procedure, a clinician can utilize a surgical stapling instrument to staple, and cut, the patient tissue. The surgical stapling instrument can include a staple cartridge that includes staples removably stored therein that are deployed into the patient tissue by a firing drive of the surgical stapling instrument. When deployed, the staples puncture a first side of the tissue and are then deformed by an anvil of the surgical stapling instrument positioned on a second, or opposite, side of the tissue. The deformed staples clench, or compress, the tissue to prevent, or at least reduce, bleeding from the incision created by the stapling instrument.

The staples can be made of a bioabsorbable material such that the staples can dissolve and release the tissue after a sufficient amount of time has elapsed following the surgical procedure. While it is desirable that the staples ultimately dissolve and release the tissue, the staples must maintain their structural integrity for an amount of time, i.e., the biocorrosion timeframe, to allow for sufficient healing of the tissue. When selecting appropriate bioabsorbable materials such that the staples can meet the biocorrosion timeframe, many factors are considered, such as the stiffness of the staples, the strength of the staples, the ductility of the staple materials, the safety of the materials being utilized (such as toxicity concerns), and/or the compatibility of the materials with electrosurgical instruments, for example. Comparatively, stents which are often implanted to hold open an artery, for example, are often comprised of alloys which resist or impede biocorrosion of the underlying structure even though a surface of the stent may comprise a dissolvable coating.

U.S. Patent No. 4,730,726 describes a sealed sterile package constructed to contain a surgical stapler having moisture-sensitive components, in particular a cartridge at a distal end which contains surgical staples made of a polymer which is subject to hydrolytic degradation. The package includes three layers. A top layer is a preformed plastic layer which is formed with at least one recess for receiving the surgical instrument. The recess is of the general contoured shape of the instrument. This layer is made of transparent material so that the packaged instrument can readily be viewed. A middle layer is a moisture permeable layer which is secured to the plastic layer about the recess. Suitable lines of glue or adhesive, such as a heat sealable adhesive, are used to secure the permeable layer directly to the plastic layer to provide a seal-tight seam. Further, the glue or adhesive which is used is such that the permeable layer is releasably secured to the plastic layer so that the permeable layer can be subsequently removed when desired, for example, by peeling of the permeable layer from the plastic layer. The permeable middle layer is made of a material which is permeable to moisture vapor and sterilant gas while being impervious to microorganisms. A bottom layer is made of a moisture impervious material such as aluminum foil. The moisture impervious layer is secured to and across the permeable layer via a line of adhesive or glue. The package also includes a desiccant pack between the permeable layer and the impervious layer. The desiccant pack is disposed adjacent to the moisture-sensitive component of the surgical instrument, on the side of the permeable layer opposite from the plastic layer and the instrument. The desiccant pack includes silica gel, molecular sieves, activated alumina or porous silica glass. In addition, the desiccant is encased within a pouch of moisture permeable polymeric material. The desiccant pack is of a size which is suitable to the size of the cartridge. The pack need not be fixedly secured in place but need only be placed on the permeable layer prior to fixing of the impervious layer to the permeable layer.

### SUMMARY

The present invention provides the packaging assembly according to claim 1 and its dependent claims. The packaging assembly comprises a surgical staple cartridge configured to be operably installed in a surgical stapling instrument, wherein the surgical staple cartridge comprises a body, a deck, a longitudinal slot defined in the deck, staple cavities defined in the deck, and staples removably stored in the staple cavities, a container configured to store the surgical staple cartridge therein and permit the surgical staple cartridge to be removed therefrom, and a desiccant element. The desiccant element is removably attached to the deck of the surgical staple cartridge and covers the staple cavities in the cartridge body. The container is hermetically sealable with the cartridge and the desiccant element removably attached to the cartridge deck stored therein.

### BRIEF DESCRIPTION OF THE DRAWINGS

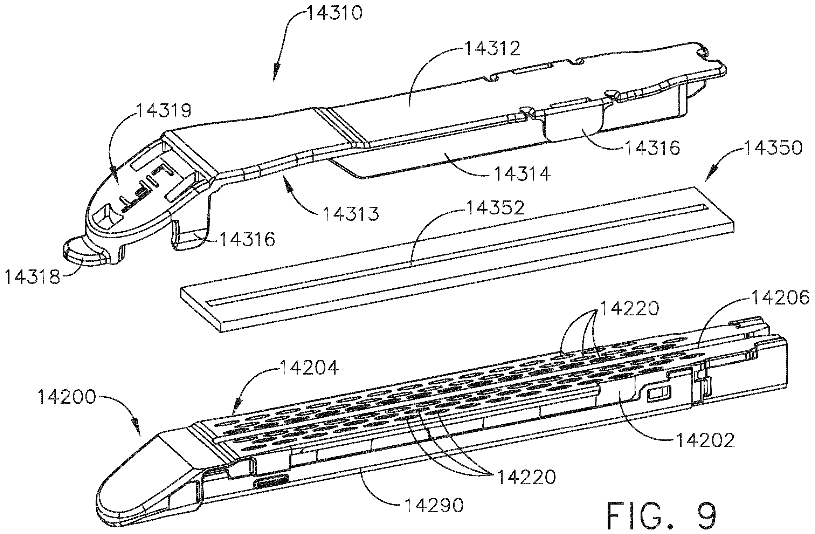

The following description is to be taken in conjunction with the accompanying drawings, in which the present invention as claimed in claim 1 is described with reference to FIGs. 9 - 12, 16, 17 and 19, and the remaining figures illustrate features of the dependent claims or alternative packaging designs, as follows:

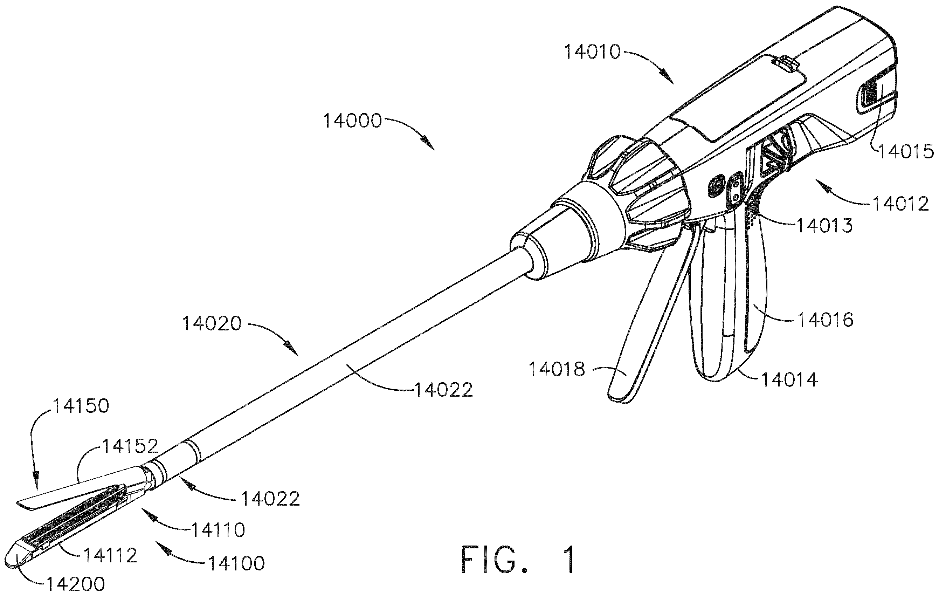

FIG. 1 is a perspective view of a surgical stapling instrument comprising a handle, a shaft assembly, and a surgical end effector;

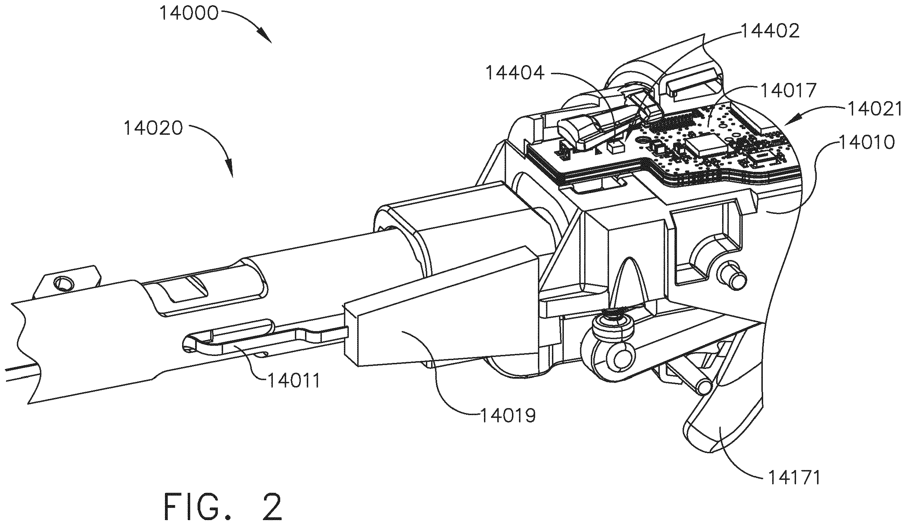

FIG. 2 is a perspective view of a portion of the shaft assembly and handle of FIG. 1;

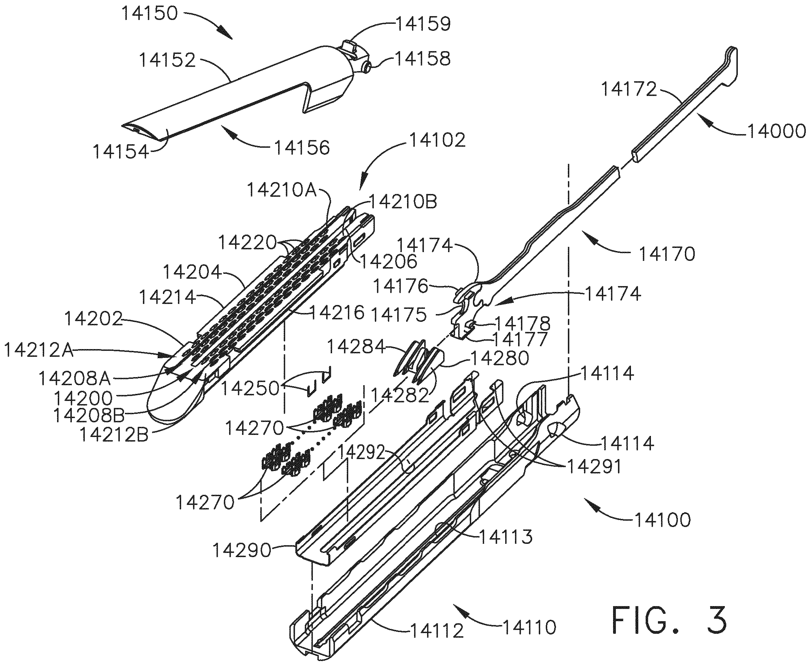

FIG. 3 is an exploded assembly view of the surgical end effector of FIG. 1;

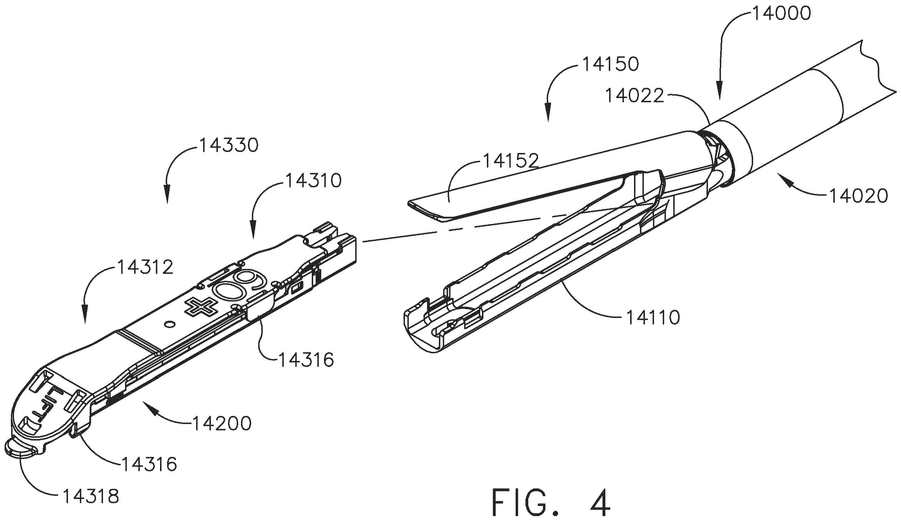

FIG. 4 is a perspective view of the surgical end effector of FIG. 1 with an anvil thereof in an open position, and wherein a cartridge/retainer assembly comprising a staple retainer attached to a surgical staple cartridge is illustrated removed from a channel of the surgical end effector;

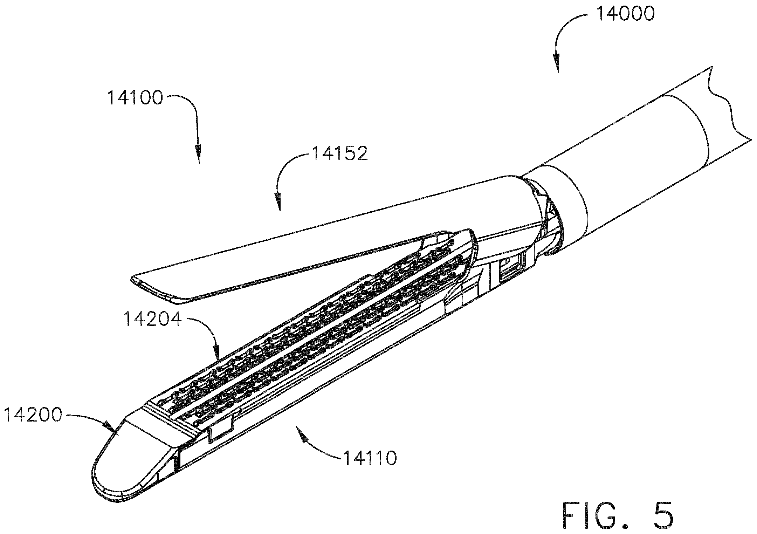

FIG. 5 is perspective view of the surgical end effector of FIG. 4, with the surgical staple cartridge of the cartridge/retainer assembly seated in the channel of the surgical end effector, and with the staple retainer removed from the surgical staple cartridge;

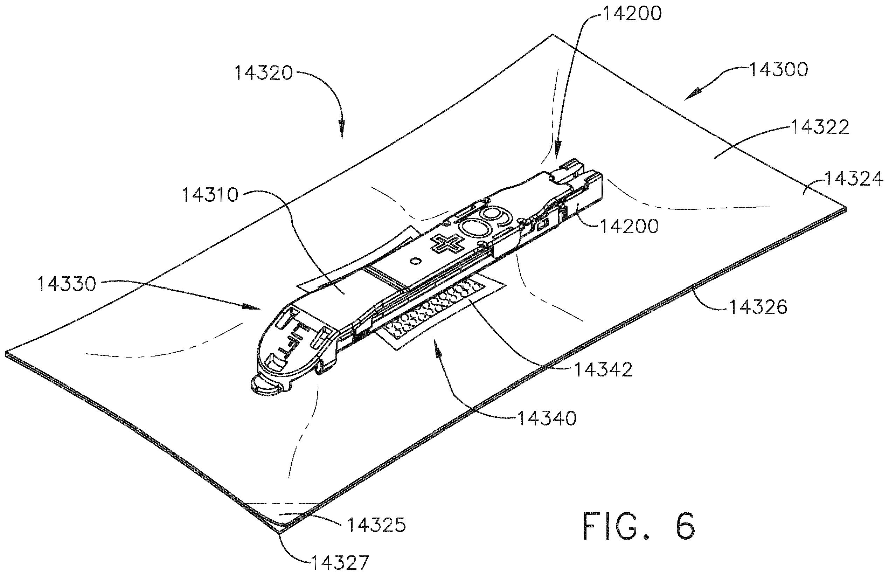

FIG. 6 is a perspective view of a packaging assembly comprising the cartridge/retainer assembly of FIG. 4 stored inside a hermetically-sealed container comprising a pouch, and wherein a desiccant element is also contained within the hermetically-sealed pouch;

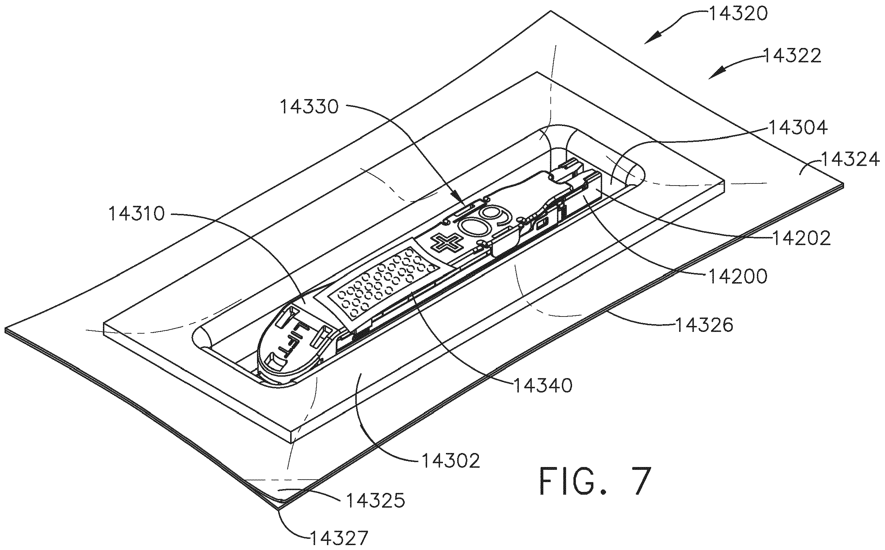

FIG.7 is a perspective view of another packaging assembly, wherein a cartridge/retainer assembly is non-movably seated in a cartridge tray that is stored within a hermetically-sealed container comprising a pouch, and wherein a desiccant element is also contained within the hermetically-sealed pouch;

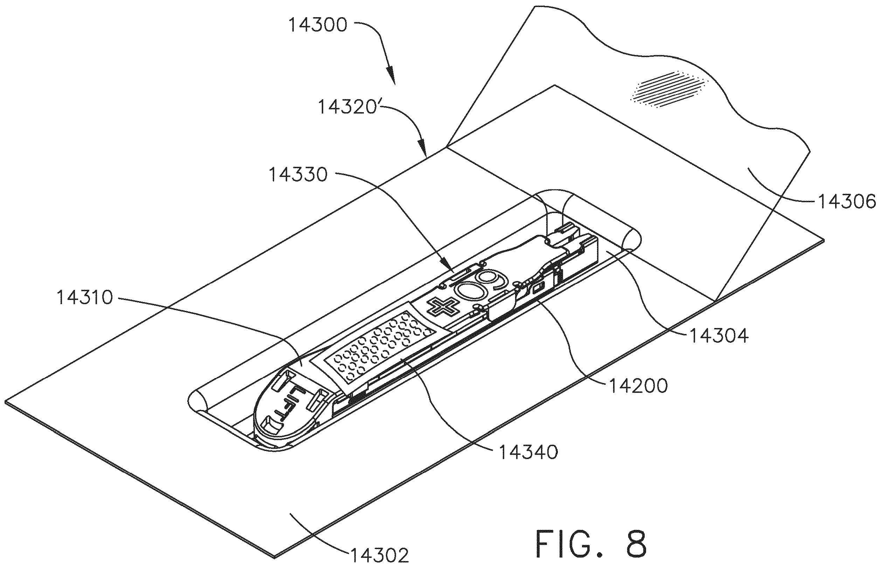

FIG. 8 is a perspective view of another packaging assembly, wherein a cartridge/retainer assembly is non-movably seated in a cartridge tray component of a hermetically-sealable container and the container further comprises a top member in a partially opened position that is attached to the cartridge tray and configured to establish a hermetic seal therewith, and wherein the container further includes a desiccant element therein;
FIG. 9 is an exploded assembly view of a staple retainer, a desiccant element, and a surgical staple cartridge, wherein the desiccant element is positioned between the staple retainer and the surgical staple cartridge;

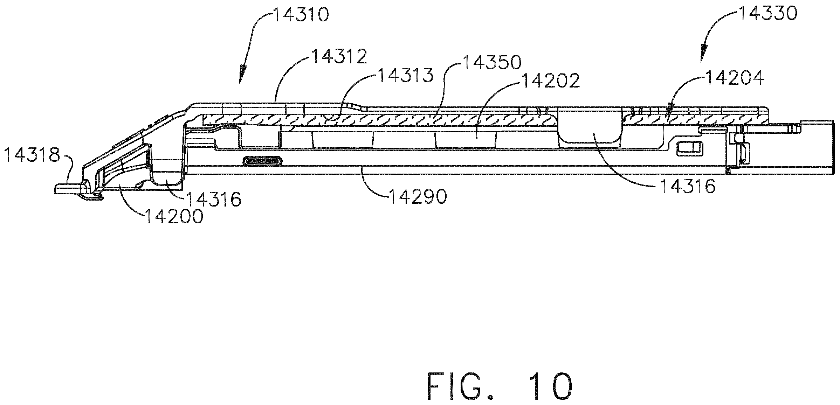

FIG. 10 is a side view of the staple retainer of FIG. 9 coupled to the surgical staple cartridge, and wherein the desiccant element of FIG. 9 is captured between the staple retainer and a deck of the surgical staple cartridge to form a cartridge/retainer assembly;

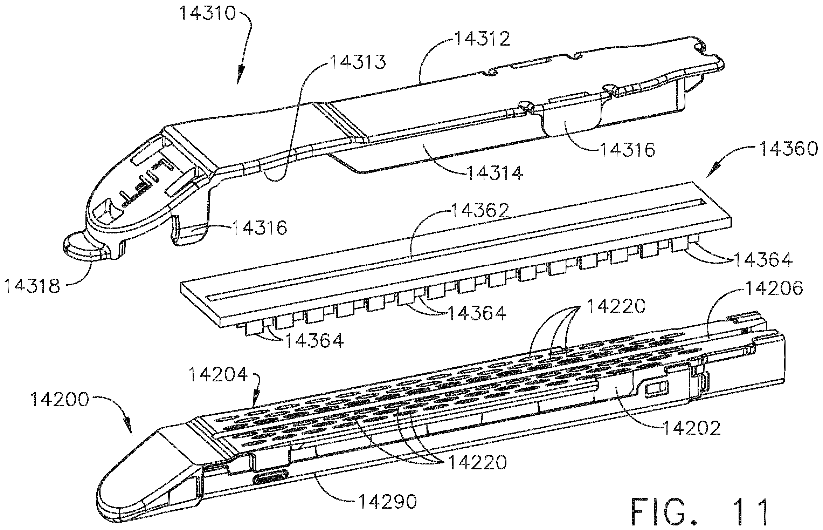

FIG. 11 is an exploded assembly view of the staple retainer of 9, wherein another desiccant element is positioned between the staple retainer and a surgical staple cartridge, wherein the desiccant element is formed with a plurality of staple retention protrusions configured to be inserted into corresponding staple cavities in the surgical staple cartridge to restrain the staples therein;

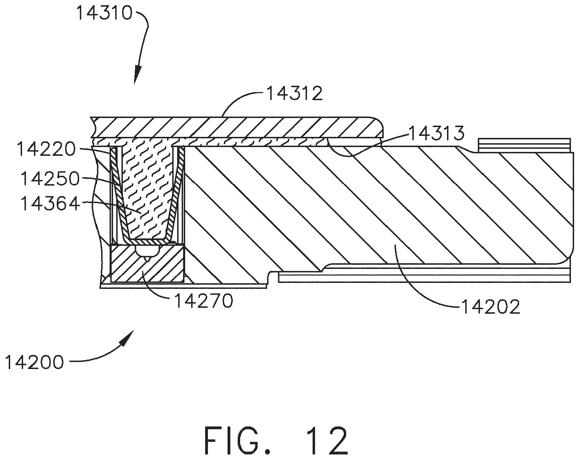

FIG. 12 is a cross-sectional view of a portion of the staple retainer of FIG. 11 coupled to the surgical staple cartridge of FIG. 11, wherein the desiccant element of FIG. 11 is captured between the staple retainer and a deck of the surgical staple cartridge, and wherein a staple retention protrusion of the staple retainer is received within a corresponding staple cavity of the surgical staple cartridge to restrain the staple contained therein on a corresponding staple driver;

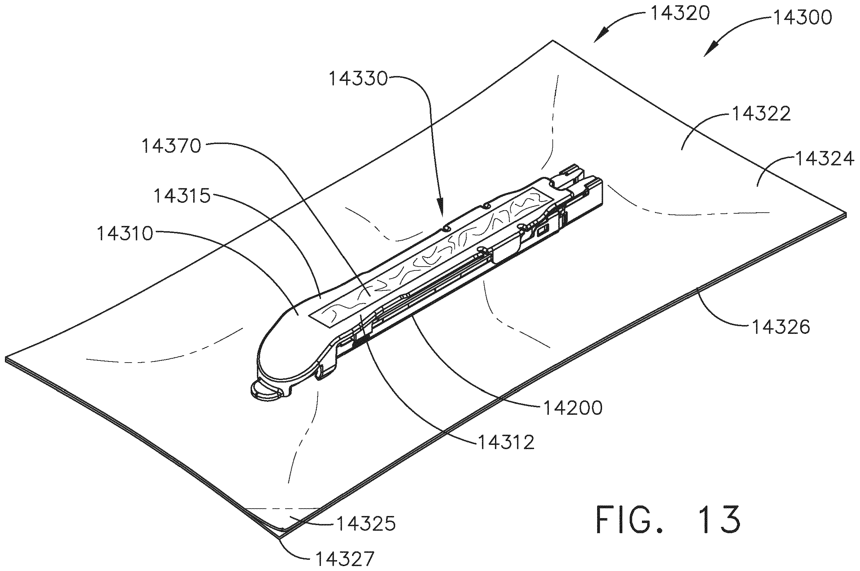

FIG. 13 is a perspective view of another packaging assembly comprising a cartridge/retainer assembly stored inside a hermetically-sealed container comprising a pouch, wherein the cartridge/retainer assembly comprises a staple retainer coupled to a surgical staple cartridge, and wherein a desiccant element is attached to the staple retainer;

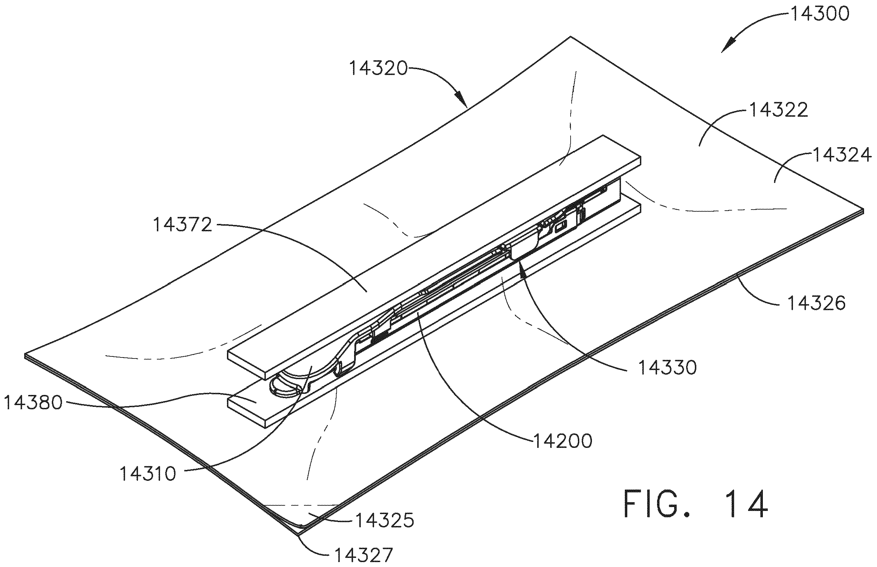

FIG. 14 is a perspective view of another packaging assembly comprising a cartridge/retainer assembly stored inside a container comprising a hermetically-sealed pouch, wherein the cartridge/retainer assembly comprises a staple retainer coupled to a surgical staple cartridge, and wherein the cartridge/retainer assembly is received between an upper desiccant element and a lower desiccant element supported within the hermetically-sealed pouch;

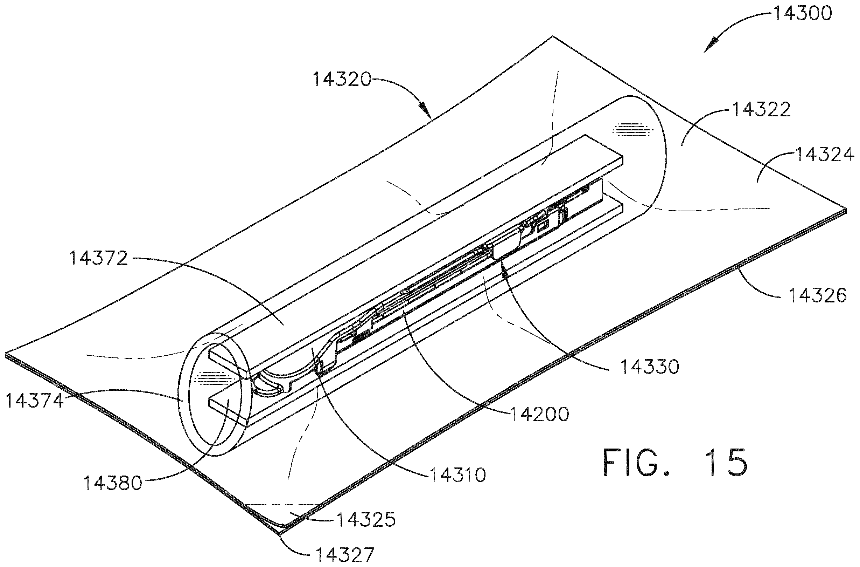

FIG. 15 is a perspective view of another packaging assembly, wherein the cartridge/retainer assembly of FIG. 14 and the upper desiccant element and lower desiccant element of FIG. 14 are contained within a retainer tube stored within a container comprising a hermetically-sealed pouch;

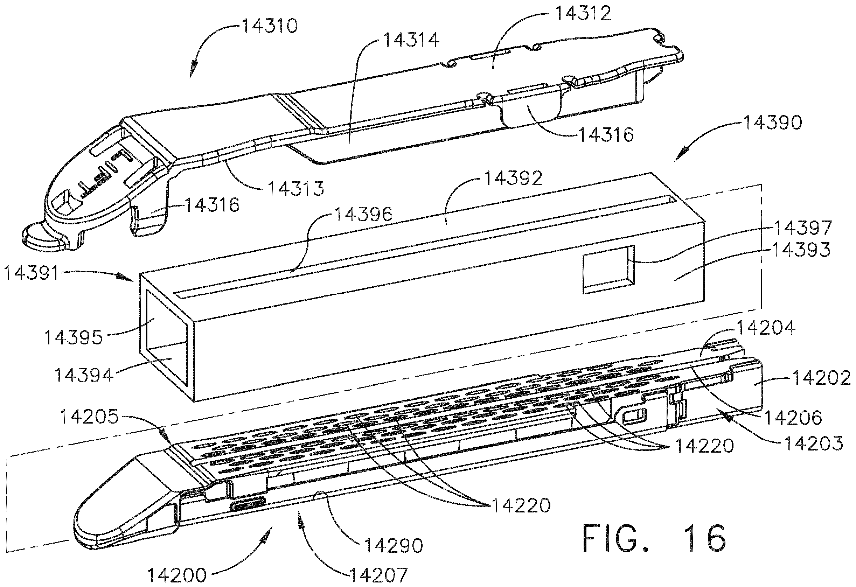

FIG. 16 is an exploded assembly view of a staple retainer, a tubular desiccant element, and a surgical staple cartridge wherein the surgical staple cartridge is to be received within the tubular desiccant element and the staple retainer is to be attached to the surgical staple cartridge to capture a portion of the tubular desiccant element between the staple retainer and a deck surface of the surgical staple cartridge;

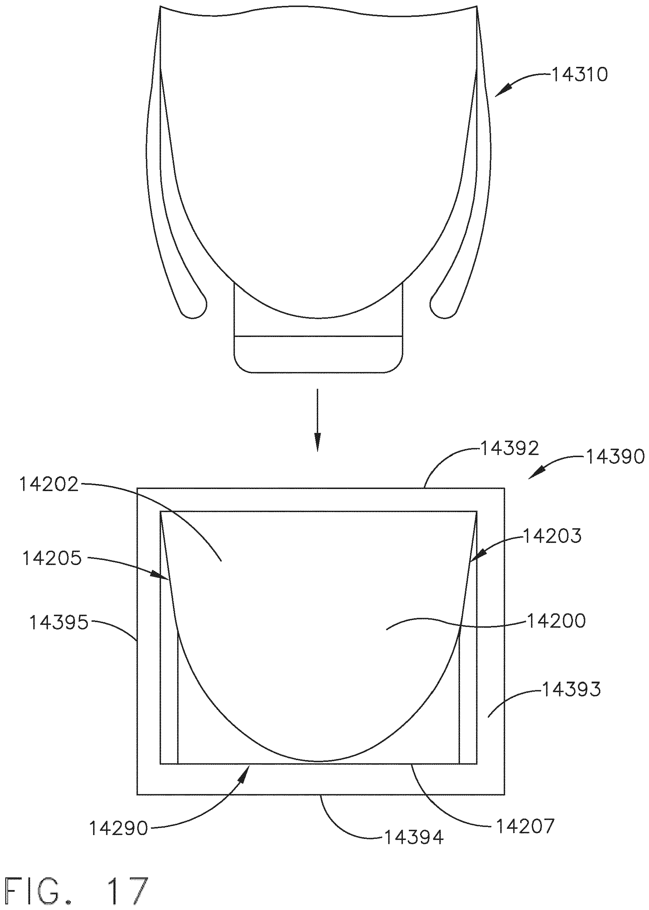

FIG. 17 is an end exploded view of the staple retainer, tubular desiccant element, and surgical staple cartridge of FIG. 16, and wherein the surgical staple cartridge has been inserted into the tubular desiccant element;

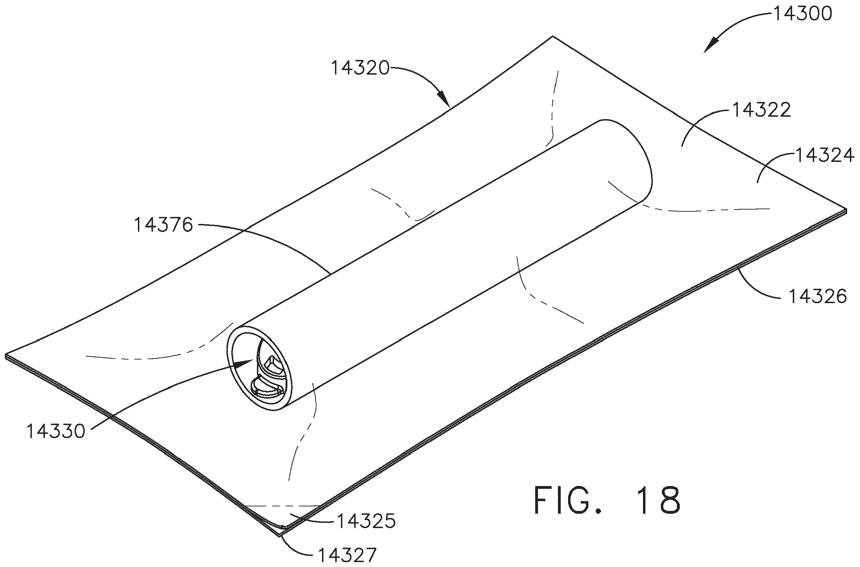

FIG. 18 is a perspective view of another packaging assembly comprising a staple retainer attached to a surgical staple cartridge to form a cartridge/ retainer assembly, wherein the cartridge/retainer assembly is received within a tubular desiccant element and is stored inside a container comprising a hermetically-sealed pouch;

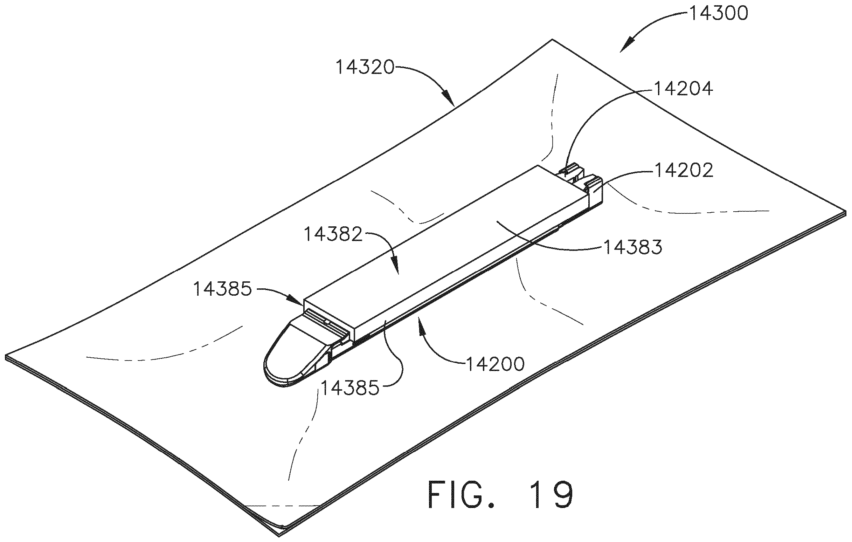

FIG. 19 is a perspective view of another desiccant element, wherein the desiccant element is removably mounted to a deck surface of a surgical staple cartridge, and wherein the surgical staple cartridge is stored in a hermetically-sealed container;

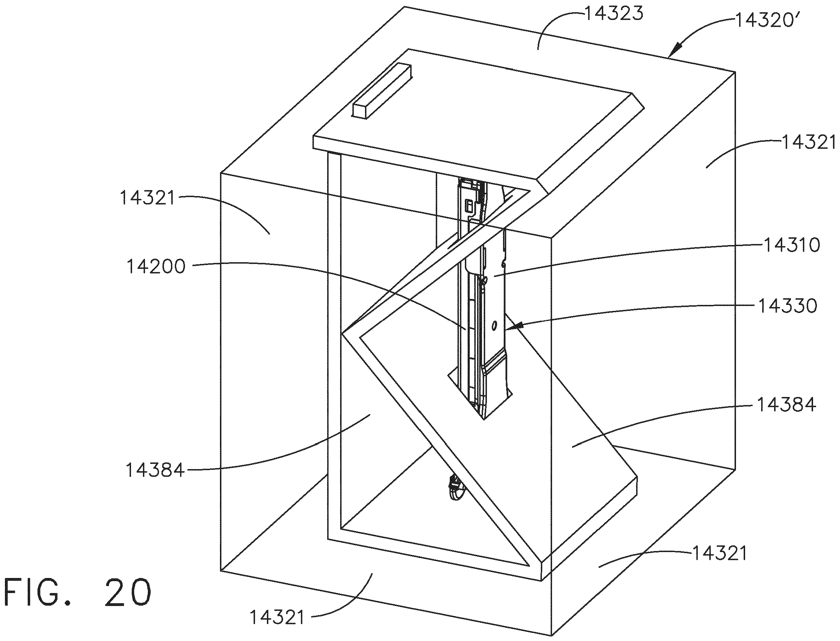

FIG. 20 is a perspective view of another desiccant element that is configured to non-movably support a cartridge/retainer assembly within a hermetically-sealable container for storage and shipment purposes;

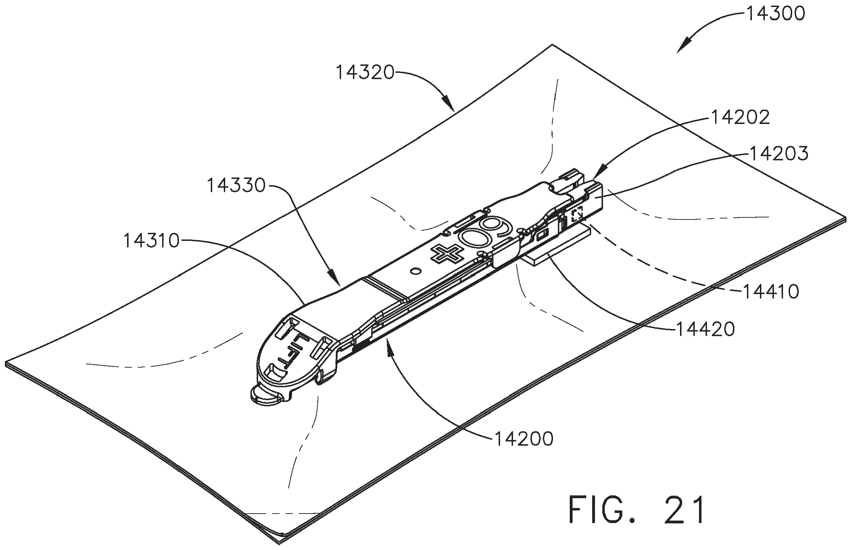

FIG. 21 is a perspective view of another packaging assembly comprising a staple retainer attached to a surgical staple cartridge to form a cartridge/ retainer assembly, wherein the cartridge/retainer assembly is stored inside a container comprising a hermetically-sealed pouch, and wherein the surgical staple cartridge comprises an RFID chip associated with a sensor;

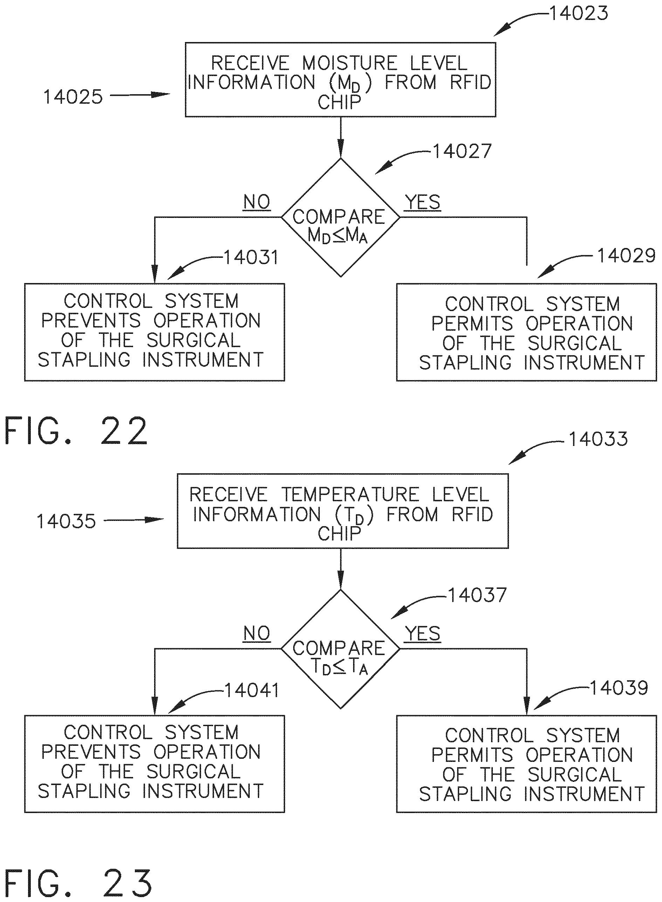

FIG. 22 is a flow chart representative of a process of controller of a surgical stapling instrument, wherein a sensor communicating with the controller monitors an amount of moisture experienced by the surgical staple cartridge of FIG. 21 while stored within the hermetically-sealed pouch of FIG. 21, and wherein the controller prevents operation of the surgical stapling instrument when a detected amount of moisture exceeds a predetermined acceptable moisture level;
FIG. 23 is a flow chart representative of another process of controller of a surgical stapling instrument, wherein a sensor communicating with the controller monitors an amount of temperature experienced by the surgical staple cartridge of FIG. 21 while stored within the hermetically-sealed pouch of FIG. 21, and wherein the controller prevents operation of the surgical stapling instrument when a detected amount of temperature exceeds a predetermined acceptable temperature level;

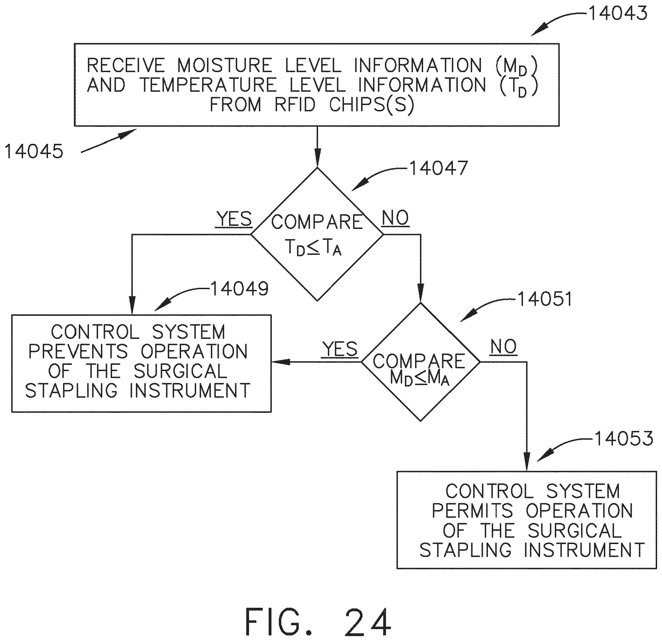

FIG. 24 is a flow chart representative of another process of controller of a surgical stapling instrument, wherein a sensor communicating with the controller monitors an amount of moisture and temperature experienced by the surgical staple cartridge of FIG. 21 while stored within the hermetically-sealed pouch of FIG. 21, and wherein the controller prevents operation of the surgical stapling instrument when a detected amount of moisture exceeds a predetermined moisture level and/or a detected amount of temperature exceeds a predetermined temperature level;

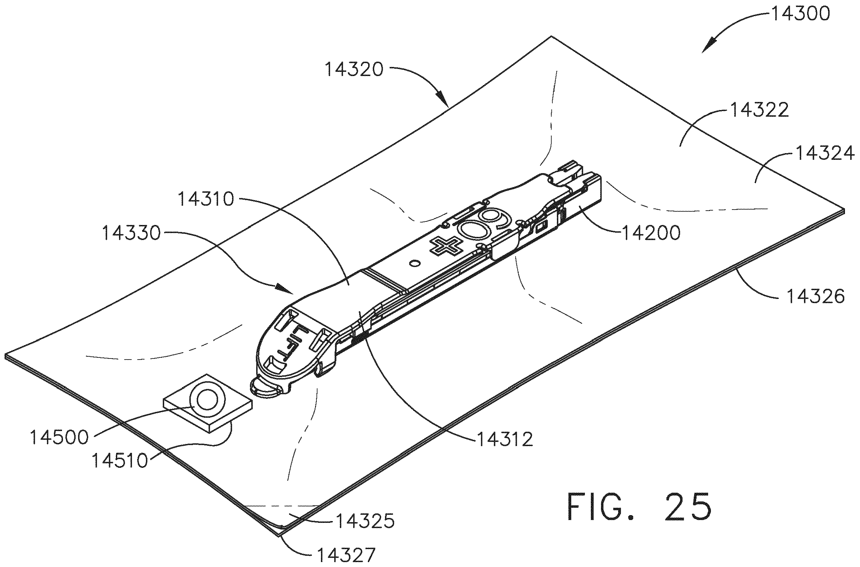

FIG. 25 is a perspective view of another packaging assembly comprising a staple retainer attached to a surgical staple cartridge to form a cartridge/ retainer assembly, wherein the cartridge/retainer assembly is stored inside a container comprising a hermetically-sealed pouch, and wherein the pouch comprises an indicator associated with a sensor;

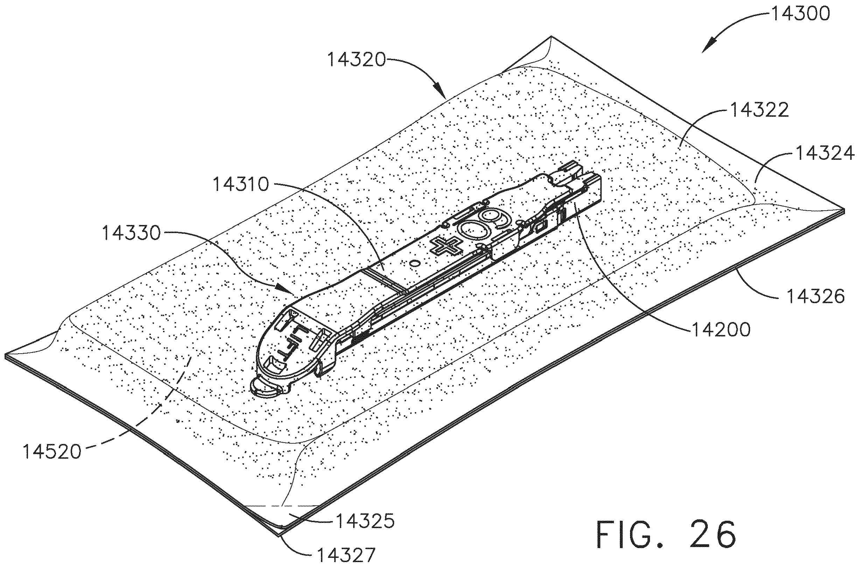

FIG. 26 is a perspective view of another packaging assembly comprising a staple retainer attached to a surgical staple cartridge to form a cartridge/ retainer assembly, wherein the cartridge/retainer assembly is stored inside a container comprising a hermetically-sealed pouch, and wherein the pouch is filled with a Nitrogen or Argon gas;
Corresponding reference characters indicate corresponding parts throughout the several views. The exemplifications set out herein are not to be construed as limiting the scope of the invention in any manner.

### DETAILED DESCRIPTION

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices are provided for performing laparoscopic and minimally invasive surgical procedures. However, the reader will readily appreciate that the various devices disclosed herein can be used in numerous surgical procedures and applications including, for example, in connection with open surgical procedures. The reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other possibilities are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other possibilities are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other possibilities are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

FIGS. 1-4 depict a surgical stapling instrument 14000 that is configured to cut and staple tissue of a patient. The surgical stapling instrument 14000 comprises a handle assembly 14010, a shaft assembly 14020 attached to the handle assembly 14010, and a surgical end effector 14100. The handle assembly 14010 comprises a housing 14012 that is configured to house various components therein such as, for example, electronics, motors, and/or drive train components. The handle assembly 14010 comprises a pistol grip portion 14014 comprising a handle 14016 that is configured to be held by a user, a closure trigger 14018 that is configured to clamp tissue within the surgical end effector 14100, and a firing trigger 14171 (FIG. 2) located forward of the closure trigger 14018 and which controls actuation of a firing system that is configured to cut and staple tissue that is clamped within the surgical end effector 14100. The handle assembly 14010 further comprises a plurality of actuators and/or buttons 14013 that are configured to electronically actuate various functions of the surgical stapling instrument 14000.

In at least one instance, the handle assembly 14010 comprises a plurality of motors positioned therein that are configured to drive one or more functions of the surgical stapling instrument 14000. The handle assembly 14010 further comprises one or more power sources such as, for example, batteries 14015 that are configured to power onboard electronics or control system 14021 that comprises, for example, the printed circuit boards 14017, 14019 which control the motor(s) positioned within the handle assembly 14010. See FIG. 2. In at least one instance, the handle assembly 14010 comprises one or more onboard memories, processors, and/or control circuits that are configured to analyze sensor data and/or control various electronic systems of the surgical stapling instrument 14000 such as, for example, motor control programs. The handle assembly 14010 may be in wireless communication with a surgical hub and/or various other components of a surgical operating suite to communicate various data between the handle assembly 14010 and the surgical hub, for example.

In the illustrated arrangement, the shaft assembly 14020 is attached to the handle assembly 14010. In at least one instance, the shaft assembly 14020 is modular and can be replaced with another shaft assembly of another surgical instrument attachment, for example. In at least one instance, the shaft assembly 14020 comprises one or more of the printed circuit boards 14017, 14019. The shaft assembly 14020 is configured to house a plurality of components of the surgical stapling instrument 14000 such as, for example, drive shafts, electronics, sensors, wires, and/or frame components, for example. Such components are configured to be coupled to corresponding components that are positioned within the handle assembly 14010 such as, for example, motors, supply leads, wires, and/or drive train components, for example. The shaft assembly 14020 houses such components and transfers such components to the surgical end effector 14100 to drive various functions of the shaft assembly 14020 and/or surgical end effector 14100 and/or transfer electrical signals between the shaft assembly 14020 and the surgical end effector 14100 and to/from the handle assembly 14010, for example. The shaft assembly 14020 comprises electrical leads 14011 electrically coupled with one or more of the printed circuit board's 14017, 14019 and one or more components within the shaft assembly 14020 and/or the surgical end effector 14100. Further details regarding the surgical stapling instrument 14000 may be found in U.S. Patent Application Serial No. 17/513,690, filed October 28, 2021, entitled ELECTRICAL LEAD ARRANGEMENTS FOR SURGICAL INSTRUMENTS.

In one form, the surgical end effector 14100 comprises a first jaw 14110 and a second jaw 14150 that is movable relative to the first jaw 14110 to grasp and ungrasp tissue therebetween. The first jaw 14110 comprises a cartridge-receiving channel 14112 that is configured to receive a surgical staple cartridge 14200 therein. The second jaw 14150 comprises an anvil 14152 that is configured to clamp onto tissue upon actuation of the closure trigger 14018 and form staples removably stored within the surgical staple cartridge 14200 upon actuation of the firing trigger 14171. As discussed above, the surgical stapling instrument 14000 may comprise various electronics. Such electronics may be wireless, wired, passively powered, and/or actively powered, for example. In various instances, such electronics may be positioned within the surgical staple cartridge 14200, on one or more components of the surgical end effector 14100 such as the cartridge-receiving channel 14112 and/or the anvil 14152, within the shaft assembly 14020, and/or within the handle assembly 14010.

A surgical staple cartridge 14200 comprises a cartridge body 14202 that defines a deck surface 14204. A longitudinal slot 14206 splits the deck surface 14204 and is configured to receive a tissue-cutting knife or E-beam member 14174 that has a tissue-cutting surface 14175 thereon. The E-beam member 14174 comprises a portion of a firing assembly 14170. See FIG. 3. The deck surface 14204 further defines longitudinal rows of staple cavities 14220 in the cartridge body 14202 located on both sides of the longitudinal slot 14206. For instance, a surgical staple cartridge 14200 can comprise three longitudinal rows 14208A, 14210A, 14212A of staple cavities 14220 on a first side 14214 of the longitudinal slot 14206 and three longitudinal rows 14208B, 14210B, 14212B of staple cavities 14220 on a second, or opposite, side 14216 of the longitudinal slot 14206. On the first side 14214 of the longitudinal slot 14206, in some cases, the longitudinal rows of staple cavities 14220 are arranged in an inner row 14208A adjacent the longitudinal slot 14206, an intermediate row 14210A adjacent the inner row 14208A, and an outer row 14212A adjacent the intermediate row 14210A. Similarly, on the second side 14216 of the longitudinal slot 14206, the longitudinal rows of staple cavities 14220 are arranged in an inner row 14208B adjacent the longitudinal slot 14206, an intermediate row 14210B adjacent the inner row 14212B, and an outer row 14212B adjacent the intermediate row 14210B.

A staple 14250 is positioned in each staple cavity 14220. Each staple 14250 is supported on a corresponding staple driver 14270 that is slidably supported with the staple cavity 14220. The staples 14250 that are positioned in the inner rows, intermediate rows, and outer rows may be comprised of the same material. The staples 14250 may comprise any of the various bio-absorbable staple compositions/configurations disclosed herein. In at least one such instance, all of the staples 14250 in the surgical staple cartridge 14200 are comprised of the same alloy, for example. In still other instances, each staple 14250 may comprise its own integrally-formed driver.

In at least one arrangement, the staples 14250 are driven from unfired positions within the cartridge body 14202 to fired positions by a firing member, such as sled 14280, for example. The sled 14280 comprises wedges 14282 which are configured to directly engage the staple drivers 14270 or the driver portion of the staples (whichever the case may be) and lift the staple drivers 14270 and staples 14250 toward the anvil 14152. The sled 14280 comprises a wedge, or rail, 14282 that corresponds to each longitudinal row of staples 14250; however, the sled 14280 may have any suitable number of wedges 14282. Each wedge 14282 comprises an angled drive surface 14284 which slides under the staple drivers 14270 or staples as the sled 14280 is advanced from a proximal end of the surgical staple cartridge 14200 toward a distal end of the surgical staple cartridge 14200.

In various arrangements, the staple drivers 14270 may be installed in the staple cavities 14220 in the cartridge body 14202 and the sled 14280 may be positioned in the cartridge body 14202 through openings the bottom of the cartridge body 14202. A metal pan 14290 may be attached to the cartridge body 14202 by tabs 14291 formed therein that are configured to retainingly engage the cartridge body 14202. The metal pan 14290 serves to retain the staple drivers 14270 and the sled 14280 within the cartridge body 14202. Each staple cavity 14220 opens through the deck surface 14204. The staples 14250 may be inserted onto their respective driver 14270 through the staple cavity opening in the deck surface 14204 or they may be installed with their corresponding staple driver through the bottom of the cartridge body 14202, for example.

In the illustrated example, the anvil 14152 comprises an elongate anvil body 14154 that has a staple-forming undersurface 14156. The staple-forming undersurface 14156 may comprise a series of staple-forming pockets that correspond to each staple 14250 in a corresponding surgical staple cartridge 14200 that is operably seated in the cartridge-receiving channel 14112. The anvil body 1454 further comprises a pair of anvil trunnions or pivot pins 14158 that are pivotally received in corresponding slots 14114 in the channel 14112 to facilitate pivotal travel of the anvil 14152 relative to the channel 14112 between an open position and a closed position.

A variety of methods and components exist for applying opening and closing motions to the anvil 14152. In one instance, for example, such opening and closing motions are applied to the anvil 14152 by an axially moving closure member or closure tube 14022. For example, the closure tube 14022 may be moved distally by compressing the closure trigger 14018. After the firing stroke has been completed, the closure tube 14022 may be moved proximally back to the starting position by releasing the closure trigger 14018 which may actuate the closure system in reverse. As the closure tube moves proximally, the closure tube 14022 may interact with an anvil opening feature or tab 14159 on the anvil 14152 to apply opening motions thereto. In addition, a spring (not shown) may be employed to apply opening motions to the anvil 14152.

The sled 14280 is configured to be actuated through a firing stroke by the firing assembly 14170 to deploy the staples 14250 from the surgical staple cartridge 14200 and cut tissue that is clamped between the jaws 14100, 14150. In various arrangements, the firing assembly 14170 may comprise an axially movable firing beam 14172 that interfaces with a firing system within the housing 14012. Once the anvil 14152 has been closed, actuation of a firing trigger 14171 causes the firing beam 14172 to move distally. The firing beam 14172 is coupled to the firing member 14174 that has a tissue-cutting surface 14175 thereon. In addition, the firing member 14174 further comprises a pair of anvil engaging tabs 14176 that are configured to be slidably received within corresponding slots in the anvil 14152. Stated another way, the anvil engaging tabs 14176 are configured to slidably engage ledges or shoulders (not shown) in the anvil 14152 and serve to maintain the staple-forming undersurface 14156 of the anvil 14152 at a desired spacing relative to the surgical staple cartridge 14200 during the firing stroke. The firing member 14174 extends through an elongate slot 14292 in the metal pan 14290 and a slot 14113 in the channel 14112. The firing member 14174 further comprises a pair of channel-engaging tabs 14177 that are located on the bottom of the firing member 14174. In addition, the firing member 14174 may comprise a pair of laterally-extending central tabs 14178 configured to slidably engage portions of the cartridge body 14202. As the firing member 14174 is driven distally during the firing stroke, the firing member 14174 contacts the sled 14280 and drives the sled 14280 distally. As the sled 14280 moves distally, the angled drive surface 14284 on each wedge 14282 cammingly interfaces with the corresponding staple drivers 14270 and causes the staple drivers 14270 to move toward the anvil 14152 which causes the staples 14250 to be ejected from the surgical staple cartridge 14200 into forming contact with the staple-forming undersurface 14156 of the anvil 14152.

In those instances wherein the staples 14250 are designed to be bio-absorbable within a desired period of time, it is desirable for the staples 14250 to be protected from physical and environmental influences that might cause premature degradation of the unfired staples 14250 during shipping and storage of the surgical staple cartridge 14200. FIG. 6 illustrates one form of a packaging assembly, generally designated as 14300, for packaging, storing, and shipping a sterile surgical staple cartridge 14200 that operably stores staples 14250 that comprise any of the various bio-absorbable staples disclosed herein.

For example, each of the staples 14250 in the surgical staple cartridge 14200 or at least some of the staples 14250 in the surgical staple cartridge 14200 may comprise a zinc-based alloy that is configured to accelerate corrosion of the staples. In another instance, each of the staples 14250 in the surgical staple cartridge 14200 or at least some of the staples 14250 in the surgical staple cartridge 14200 may comprise a magnesium-based alloy and be coated with a coating comprised of an absorbable polymer and a calcification inhibitor. In still another instance, for example, each of the staples 14250 in the surgical staple cartridge 14200 or at least some of the staples 14250 in the surgical staple cartridge 14200 may be hollow and comprise a magnesium-based alloy. In yet another example, each of the staples 14250 in the surgical staple cartridge 14200 or at least some of the staples 14250 in the surgical staple cartridge 14200 may comprise a magnesium-based alloy, wherein an alloying element of the magnesium-based alloy is selected to lower an electrode potential of the staples. In yet another arrangement, each of the staples 14250 in the surgical staple cartridge 14200 or at least some of the staples 14250 in the surgical staple cartridge 14200 may comprise a magnesium-based alloy, wherein an alloying element of the magnesium-based alloy is selected to accelerate anodic corrosion of magnesium of the magnesium-based alloy. In another arrangement, at least a portion of each staple 14250 in the surgical staple cartridge 14200 or at least some of the staples 14250 in the surgical staple cartridge 14200 is coated in Fetuin A. In other instances, at least a portion of each staple 14250 in the surgical staple cartridge 14200 or at least some of the staples 14250 in the surgical staple cartridge 14200 is coated by one or more proteins. In other cases, at least a portion of each staple 14250 in the surgical staple cartridge 14200 or at least some of the staples 14250 in the surgical staple cartridge 14200 is coated in citrate. In another arrangement, at least a portion of each staple 14250 in the surgical staple cartridge 14200 or at least some of the staples 14250 in the surgical staple cartridge 14200 is coated in a chelating agent. In another arrangement, at least a portion of each staple 14250 in the surgical staple cartridge 14200 or at least some of the staples 14250 in the surgical staple cartridge 14200 is coated in phytic acid. In various alternatives, at least a portion of each staple 14250 in the surgical staple cartridge 14200 or at least some of the staples 14250 in the surgical staple cartridge 14200 is coated in pyrophosphate. In another arrangement, at least a portion of each staple 14250 in the surgical staple cartridge 14200 or at least some of the staples 14250 in the surgical staple cartridge 14200 is coated in a bisphosphonate. In still other arrangements, at least a portion of each staple 14250 in the surgical staple cartridge 14200 or at least some of the staples 14250 in the surgical staple cartridge 14200 is coated in a polyphosphate. Alternatively, at least a portion of each staple 14250 in the surgical staple cartridge 14200 or at least some of the staples 14250 in the surgical staple cartridge 14200 is coated in a co-polymer of acrylic acid. In other cases, at least a portion of each staple 14250 in the surgical staple cartridge 14200 or at least some of the staples 14250 in the surgical staple cartridge 14200 is coated in a polycarboxylic acid. In another instance, at least a portion of each staple 14250 in the surgical staple cartridge 14200 or at least some of the staples 14250 in the surgical staple cartridge 14200 is coated in a polymer coating. In another arrangement, at least a portion of each staple 14250 in the surgical staple cartridge 14200 or at least some of the staples 14250 in the surgical staple cartridge 14200 is coated in osteopontin. In still another arrangement, at least a portion of each staple 14250 in the surgical staple cartridge 14200 or at least some of the staples 14250 in the surgical staple cartridge 14200 is coated in magnesium ions.

After the staples 14250 have been installed in the surgical staple cartridge 14200, the surgical staple cartridge 14200 may then be sterilized utilizing, for example, gamma radiation, x-rays, high-energy electrons, beta radiation, ethylene oxide, plasma peroxide, steam etc. In one arrangement, the packaging assembly 14300 comprises a staple retainer 14310 that is removably coupled to the surgical staple cartridge 14200 to form a cartridge/retainer assembly 14330. See FIG. 4. When coupled to the surgical staple cartridge 14200, the staple retainer 14310 covers the staple cavities 14220 and serves to retain the staples 14250 within each staple cavity 14220 during shipping and storage of the surgical staple cartridge 14200. The staple retainer 14310 is configured to be removed from the surgical staple cartridge 14200 prior to use of the surgical staple cartridge 14200. For example, the staple retainer 14310 may be removed from the surgical staple cartridge 14200 prior to installation in the cartridge-receiving channel 14112 or the cartridge/retainer assembly 14330 may be installed in the channel 14112 and thereafter, the staple retainer 14310 is removed prior to use of the surgical stapling instrument 14000. See FIG. 5. The staple retainer 14310 may be separately sterilized prior to installation on the surgical staple cartridge 14200 or the staple retainer 14310 may be installed onto the surgical staple cartridge 14200 and the entire cartridge/retainer assembly 14330 may then be sterilized. After sterilization, the cartridge/retainer assembly 14330 is then placed into a hermetically-sealed (airtight and fluidtight) sterile container 14320 for shipping and storage. See FIG. 6.

One form of a staple retainer 14310 may be fabricated from a polymer material and comprise an elongate body portion 14312 that is configured to be received on the deck surface 14204 of the surgical staple cartridge 14200. See FIG. 9. The elongate body portion 14312 is sized relative to the deck surface 14204 such that when the staple retainer 14310 is received thereon, the elongate body portion 14312 covers all of the staple cavities 14220 in the cartridge body 14202. In various instances, the body portion 14312 of the staple retainer 14310 may be formed with instructional indicia 14319 and/or identifying indicia that relates to a particular surgical staple cartridge. To facilitate positioning of the staple retainer 14310 on the deck 14204, a downwardly extending locator keel or locator member 14314 may protrude downward from a deck-facing surface 14313 of the elongate body portion 14312 and is configured to be received within the elongate slot 14206 in the cartridge body 14202. See FIG. 9. In at least one arrangement, the staple retainer 14310 is formed with a plurality of attachment tabs 14316 that are configured to retainingly engage corresponding portions of the cartridge body 14202 to removably affix the staple retainer 14310 to the cartridge body 14202. In at least one instance, the staple retainer 14310 is configured to prevent the staples 14250 from falling out of their respective staple cavities 14220 during shipping and/or installation of the surgical staple cartridge 14200 into the channel 14112 of the surgical stapling instrument 14000 as well as prevents infiltration of debris into the staple cavities 14220 prior to use.

The staple retainer 14310 is configured to be removed prior to use of the surgical stapling instrument 14000 and the surgical staple cartridge 14200. To facilitate such removal, the staple retainer 14310 may comprise a removal tab 14318 that protrudes from a distal end of the staple retainer 14310 to facilitate prying of the staple retainer 14310 from the cartridge body 14202 of the surgical staple cartridge 14200. Various other staple retainer arrangements are disclosed in U.S. Patent No. 11,185,330, entitled FASTENER CARTRIDGE ASSEMBLIES AND STAPLE RETAINER COVER ARRANGEMENTS, and U.S. Patent No. 9,839,420, entitled TISSUE THICKNESS COMPENSATOR COMPRISING AT LEAST ONE MEDICAMENT.

The packaging assembly 14300 further comprises a hermetically-sealable (airtight and fluidtight) container 14320 that is configured to temporarily store one or more cartridge/retainer assemblies 14330 therein prior to use. See FIG. 6. Such hermetically-sealable container 14320 is configured to store and protect the cartridge/retainer assemblies 14330 after being manufactured and sterilized until the container 14320 is opened in the operating room, for example. As described in U.S. Patent Application Publication No. 2020/0405296, entitled PACKAGING FOR A REPLACEABLE COMPONENT OF A SURGICAL STAPLING SYSTEM, various container arrangements may comprise peel-pouches, woven and/or non-woven material wrappers, rigid containers, etc.

In the examples illustrated in FIGS. 6, 7, 13, 14, 15, 18, 19, 21, 25, and 26, the container 14320 comprises a hermetically-sealable peel-pouch 14322 that is fabricated from a gas and moisture impermeable foil or plastic material. In at least one arrangement, for example, the container 14320 comprises a first layer 14324 and a second layer 14326. The first layer 14324 and the second layer 14326 form a protective barrier around a cartridge/retainer assembly 14330. An adhesive bonds the first layer 14324 and the second layer 14326 together to form an airtight and fluidtight sealed pouch around the cartridge/retainer assembly 14330. The adhesive forms a seal without creases, wrinkles, and/or gaps. The seal created by the adhesive prevents contaminants from coming into contact with the cartridge/retainer assembly 14330 as well as prevents air/gas/fluid from infiltrating into the interior of the container 14320. As discussed in U.S. Patent Application Publication No. 2020/0405296, the first layer 14324 may comprise a first corner 14325 positioned outside of the seal, and the second layer 14326 may comprise a second corner 14327 positioned outside of the seal. The clinician can expose the cartridge/retainer assembly 14330 by peeling the first layer 14324 apart from the second layer 14326. In various instances, the clinician can expose the sealed cartridge/retainer assembly 14330 by holding the first corner 14325 and the second corner 14327 in separate hands and pulling the first corner 14325 in a direction away from the second layer 14326, although any suitable opening method could be used.

The first layer 14324 and the second layer 14326 may be comprised of a material such as, for example, paper with a laminated inner surface. The laminated inner surface provides a gas impermeable and a fluid impermeable barrier to prevent contaminants from entering the sealed portion of the container 14320. In one arrangement, the first layer 14324 and the second layer 14326 are comprised of foil. In another arrangement, the first layer 14324 and the second layer 14326 are comprised of plastic. In various arrangements, the first layer 14324 and/or the second layer 14326 can be comprised of a material with a particular degree of transparency to allow a clinician, for example, to observe the contents of the container 14320. Additionally, the container 14320 may comprise any of the various identification features/indicia described in U.S. Patent Application Publication No. 2020/0405296.

In various instances, the container 14320 may be sized relative to a cartridge/retainer assembly 14330 such that when the cartridge/retainer assembly 14330 is received therein, the cartridge/retainer assembly 14330 is substantially non-movably retained in position within the container 14320. Although FIG. 6 illustrates a container 14320 that is sized to contain one cartridge/retainer assembly 14330 therein, the container 14320 may be sized to contain a plurality of cartridge/retainer assemblies 14330 or surgical staple cartridges 14200 therein. In either case, the container 14320 may be sized relative to the cartridge/retainer assemblies 14330 to substantially prevent movement therein once the container 14320 is sealed.

As discussed above, in those instances wherein the staples 14250 are designed to be bio-absorbable within a desired period of time, it is desirable for the staples 14250 to be protected from physical and environmental influences that might cause premature degradation of the unfired staples 14250 during shipping and storage of the surgical staple cartridge 14200. For example, should the staples 14250 be exposed to moisture prior to use, the staples 14250 may begin to undesirably degrade. Thus, it is desirable to prevent the staples from being exposed to moisture while being stored and shipped prior to use. In the arrangement depicted in FIG. 6, the packaging assembly 14300 further comprises a desiccant element 14340 that is sealed in the container 14320 with the cartridge/retainer assembly 14330. In one arrangement, the desiccant element 14340 comprises a pouch 14342 that contains a desiccant material such as, for example, silica gel, activated alumina, etc. that are configured to absorb and ultimately reduce the moisture within the hermetically-sealed container 14320. In still other arrangements, the desiccant element 14340 comprises 220 g/m² +/- 5% Invercote T2 paper per P-02-003 (Adaptiv) or other commercially available moisture absorbent boards.

In various instances, the desiccant element 14340 may be configured to change color in the presence of moisture. In such instance, the desiccant element 14340 would provide the clinician with an indication that moisture was present within the container 14320 and could have caused premature degradation of the bio-absorbable staples 14250 allowing the clinician to avoid use of the surgical staple cartridge 14200. For example, in at least one arrangement, the desiccant element 14340 may comprise a blue desiccant material containing cobalt chloride which will change to a pink color when the desiccant material has reached its maximum adsorption capacity. This change in color would indicate a compromised package. In another arrangement, for example, the desiccant element 14340 may comprise a PH sensitive dye that changes color upon exposure to basic environments which may indicate corrosion of Mg to Mg(OH)2. In another arrangement, the desiccant element 14340 may be treated with an irreversible hydrochromic dye such as, for example, patent blue V (tryphenylmethane) dye, cobalt II chloride or methyl violet, for example.

In other arrangements, the packaging assembly 14300 may additionally comprise a tray 14302 that is configured with a cartridge retention receptacle 14304 or other retention features that non-movably retain one or more cartridge/retainer assemblies 14330 or surgical staple cartridges 14200 in position on the tray 14302. See FIG. 7. In addition, a desiccant element 14340 may be received within the receptacle 14304 on top of the cartridge/retainer assembly 14330 or otherwise positioned therein. When the clinician is ready to use a surgical staple cartridge 14200, the container 14320 is opened and the tray 14302 is removed or otherwise positioned to allow the clinician to again access to the cartridge/retainer assembly 14330 and pry or otherwise remove the cartridge/retainer assembly 14330 out of retaining engagement within the receptacle 14304 in the tray 14302. In certain instances, the surgical staple cartridge 14200 or the cartridge/retainer assembly 14330 may be attached to the tray 14302 during the sterilization process. The tray 14302, as well as the staple retainer 14310, provides air/fluid paths for a sterilization medium to invade around the surgical staple cartridge 14200 and be flushed from the interior portions of the cartridge body 14202.

FIG. 8 illustrates an alternative packaging assembly 14300 that comprises a container 14320' that comprises a tray 14302 of the type and construction described above with a top layer 14306 removably sealed thereto so as to create a hermetic seal between the top layer 14306 and the tray 14302. In various instances, the tray may comprise a gas and moisture impermeable material and the top layer may comprise a gas and moisture impermeable foil material, for example. The top layer 14306 may be sealed to the tray 14302 by an adhesive, for example.

The packaging assembly 14300 may comprise a desiccant element 14350 that is configured to be received between the deck surface 14204 and the deck-facing surface 14313 of the staple retainer 14310. See FIG. 9. In one arrangement the desiccant element 14350 comprises a 220 g/m² +/- 5% Invercote T2 paper per P-02-003 (Adaptiv) or other commercially available moisture absorbent board that has a slot 14352 therein that is configured to accommodate insertion of the retainer keel 14314 therethrough. In another arrangement, the desiccant element 14350 comprises a compliant desiccant material. In all of the foregoing arrangements, the desiccant element 14350 is sized to cover the deck surface 14204 such that the desiccant element 14350 covers all of the staple cavities 14220. In one arrangement, the desiccant element 14350 is placed onto the deck surface 14204 and is held in place by the staple retainer 14310. That is, the desiccant element 14350 is trapped between the deck-facing surface 14313 of the staple retainer 14310 and the deck surface 14204. In such instance, the desiccant element 14350 covers each staple cavity 14220 and serves to absorb moisture occurring therein (if any). The staple retainer 14310 and the desiccant element 14350 are removed before the surgical staple cartridge 14200 can be used. In another arrangement, the desiccant element 14350 is attached to the deck-facing surface 14313 of the staple retainer 14310 with an appropriate adhesive, for example. In such arrangement, the desiccant element 14350 is removed from the surgical staple cartridge 14200 when the staple retainer 14310 is detached therefrom. The desiccant element 14350 may comprise an adhesive strip (not shown) that is configured to be removably attached to the deck surface 14204. In such arrangement, a staple retainer 14310 may or may not be removably attached to the surgical staple cartridge 14200 in the above-described manner.

In at least one arrangement, the desiccant element 14350 may comprise a blue desiccant material containing cobalt chloride which will change to a pink color when the desiccant material has reached its maximum adsorption capacity. This change in color would indicate a compromised package. In another arrangement, for example, the desiccant element 14350 may comprise a PH sensitive dye that changes color upon exposure to basic environments which may indicate corrosion of Mg to Mg(OH)2. Alternatively, the desiccant element 14350 may be treated with an irreversible hydrochromic dye such as, for example, patent blue V (tryphenylmethane) dye, cobalt II chloride or methyl violet, for example.

FIG. 11 illustrates another desiccant element 14360 that may comprise a portion of the packaging assembly 14300. As can be seen in FIG. 11, the desiccant element 14360 is configured to be received between the deck surface 14204 and the deck-facing surface 14313 of the staple retainer 14310. In one arrangement the desiccant element 14360 comprises a 220 g/m² +/- 5% Invercote T2 paper per P-02-003 (Adaptiv) or other commercially available moisture absorbent board that has a slot 14362 therein that is configured to accommodate insertion of the retainer keel 14314 therethrough. Additionally, the desiccant element 14360 is formed with a plurality of individual staple retention protrusions 14364 that protrude downward from a deck-facing surface 14362 of the desiccant element 14360. In the illustrated arrangement, each retention protrusion is 14364 is configured to protrude into a corresponding staple cavity 14220 such that each staple cavity 14220 has a corresponding staple retention protrusion 14364 protruding therein when the desiccant element 14360 is received on the deck 14204. Each staple retention protrusion 14364 is configured to non-movably retain the corresponding staple 14250 on the corresponding staple driver 14270 within the corresponding staple cavity 14220. See FIG. 12. Such desiccant element 14360 immovably restrains the staples 14250 within their respective staple cavities 14220 and prevents their movement which might damage or compromise the staple and/or staple coating.

In at least one arrangement, the desiccant element 14360 may comprise a blue desiccant material containing cobalt chloride which will change to a pink color when the desiccant material has reached its maximum adsorption capacity. This change in color would indicate a compromised package. In another arrangement, for example, the desiccant element 14360 may comprise a PH sensitive dye that changes color upon exposure to basic environments which may indicate corrosion of Mg to Mg(OH)2. Alternatively, the desiccant element 14360 may be treated with an irreversible hydrochromic dye such as, for example, patent blue V (tryphenylmethane) dye, cobalt II chloride or methyl violet, for example.

FIG. 13 illustrates use of another desiccant element 14370 that is formed as an adhesive strip that is configured to be attached to an upper surface 14315 of the staple retainer 14310. The desiccant element 14370 may comprise a blue desiccant material containing cobalt chloride which will change to a pink color when the desiccant material has reached its maximum adsorption capacity. This change in color would indicate a compromised package. In another arrangement, for example, the desiccant element 14360 may comprise a PH sensitive dye that changes color upon exposure to basic environments which may indicate corrosion of Mg to Mg(OH)2. Alternatively, the desiccant element 14360 may be treated with an irreversible hydrochromic dye such as, for example, patent blue V (tryphenylmethane) dye, cobalt II chloride or methyl violet, for example.

FIG. 14 illustrates a cartridge retainer assembly 14330 sandwiched between an upper desiccant element 14372 and a lower desiccant element 14380. The upper desiccant element 14372 and the lower desiccant element 14380 may each comprise a 220 g/m² +/- 5% Invercote T2 paper per P-02-003 (Adaptiv) or other commercially available moisture absorbent board. In other arrangements, the upper desiccant element 14372 and the lower desiccant element 14380 may each comprise a compliant desiccant material that offers some cushioning protection to the cartridge/retainer assembly 14330. Such arrangement may additionally provide the cartridge retainer assembly 14330 with cushioning and physical protection from external impacts to the container 14320. In one arrangement, the upper desiccant element 14372 may be affixed to an inner surface of the first layer 14324 of the container 14320 by an adhesive. Similarly, the lower desiccant element 14380 may be affixed to an inner surface of the second layer 14326 in registration with the upper desiccant element 14372 such that the cartridge/retainer assembly 14330 may be positioned therebetween within the container 14320. In other arrangements, the upper desiccant element 14372 may be attached to the upper surface 14315 of the staple retainer 14310 with an adhesive and the lower desiccant element 14380 may be removably attached to the bottom surface of the metal pan 14290. In such instance, the clinician would remove the staple retainer 14310 and the lower desiccant element 14380 before use. When installed in the container 14320, the upper desiccant element 14372 and the lower desiccant element 14380 may serve to substantially immovably constrain the cartridge/retainer assembly 14330 within the container 14320. In an alternative arrangement, the cartridge/retainer assembly 14330 and the upper desiccant element 14372 and lower desiccant element 14380 are inserted into a retainer tube 14374. In one arrangement, the retainer tube 14374 may be formed from a transparent polymer material that permits the cartridge/retainer assembly 14330 to be viewed through the retainer tube 14374 for identification purposes. Retainer tube 14374 serves to hold the upper desiccant element 14372 and lower desiccant element 14380 in registration with the cartridge/retainer assembly 14330 within the container 14320 and affords added protection to the cartridge/retainer assembly 14330. See FIG. 15.

In at least one arrangement, the upper desiccant element 14372 and/or the lower desiccant element 14380 may comprise a blue desiccant material containing cobalt chloride which will change to a pink color when the desiccant material has reached its maximum adsorption capacity. This change in color would indicate a compromised package. In another arrangement, for example, the upper desiccant element 14372 and/or the lower desiccant element 14380 may comprise a PH sensitive dye that changes color upon exposure to basic environments which may indicate corrosion of Mg to Mg(OH)2. Alternatively, the upper desiccant element 14372 and/or the lower desiccant element 14380 may be treated with an irreversible hydrochromic dye such as, for example, patent blue V (tryphenylmethane) dye, cobalt II chloride or methyl violet, for example.

FIGS. 16 and 17 illustrate another desiccant element 14390 that comprises a tubular-shaped member 14391 that is configured to receive a surgical staple cartridge 14200 therein. The desiccant element 14390 may comprise a 220 g/m² +/- 5% Invercote T2 paper per P-02-003 (Adaptiv) or other commercially available moisture absorbent board. For example, the desiccant element 14390 comprises a top portion 14392 that is adapted to cover the staple cavities 14220 in the deck surface 14204 when the surgical staple cartridge 14200 has been inserted therein. The top portion 14392 is attached to a bottom portion 14394 by a first side 14393 that corresponds to a first side 14203 of the cartridge body 14202 and a second side 14395 that corresponds to a second side 14205 of the cartridge body 14202 to form a tubular structure sized to receive a surgical staple cartridge 14200 therein. The bottom portion 14394 of the desiccant element 14390 may be substantially coextensive with a bottom portion 14207 of the cartridge body 14202 which is defined by the pan 14290. A slot 14396 is provided through the top portion 14392 to accommodate passage of the retainer keel 14314 therethrough. The surgical staple cartridge 14200 is inserted into the desiccant element 14390 until the slot 14396 in the top portion 14392 is in registration with the longitudinal slot 14206 in the surgical staple cartridge 14200. The staple retainer 14310 may then be attached to the cartridge/element assembly by inserting the retainer keel 13314 into the slots 14396 and 14206. The proximal-most attachment tabs 14316 are in registration with corresponding windows 14397 in the first side 14393 and the second side 14395 to engage the cartridge body 14202. The desiccant element 14390 stops short of the distal-most attachment tabs 14316 such that the distal-most attachment tabs 14316 may engage the surgical staple cartridge 14200. In other instances, the desiccant element 14390 may be used without a staple retainer 14310. In such instance, for example, the top portion 14392 would not include the longitudinal slot 14396 and the windows 14397 would not be provided in first side 14393 and the second side 14395. The surgical staple cartridge 14200 would be similarly inserted into the desiccant element 14390 for storage therein. The entire unit is then sealed within a hermetically-sealed container 14320.

The desiccant element 14390 may comprise a blue desiccant material containing cobalt chloride which will change to a pink color when the desiccant material has reached its maximum adsorption capacity. This change in color would indicate a compromised package. In another arrangement, for example, the desiccant element 14390 may comprise a PH sensitive dye that changes color upon exposure to basic environments which may indicate corrosion of Mg to Mg(OH)2. Alternatively, the desiccant element 14390 may be treated with an irreversible hydrochromic dye such as, for example, patent blue V (tryphenylmethane) dye, cobalt II chloride or methyl violet, for example.

FIG. 18 illustrates use of a desiccant element 14376 that is formed as a hollow tube that is sized to receive a cartridge/retainer assembly 14330 therein. The cartridge/retainer assembly 14330 is inserted into the desiccant element 14376 and the entire unit is inserted into the container 14320 for storage and shipping. The desiccant element 14376 may comprise a blue desiccant material containing cobalt chloride which will change to a pink color when the desiccant material has reached its maximum adsorption capacity. This change in color would indicate a compromised package. In another arrangement, for example, the desiccant element 14376 may comprise a PH sensitive dye that changes color upon exposure to basic environments which may indicate corrosion of Mg to Mg(OH)2. In another arrangement, the desiccant element 14376 may be treated with an irreversible hydrochromic dye such as, for example, patent blue V (tryphenylmethane) dye, cobalt II chloride or methyl violet, for example.

FIG. 19 illustrates another desiccant element 14382 that is supported or stored in a hermetically-sealable container 14320. In various instances, the desiccant element 14382 is configured to be removably attached to the deck surface 14204 of the surgical staple cartridge 14200. The desiccant element 14382 is sized to cover all of the staple cavities 14220 in the surgical staple cartridge 14200 and, as such, eliminates a need to employ a separate staple retainer. Stated another way, the desiccant element 14382 functions as a staple retainer while also providing an additional advantage of moisture absorption. In one arrangement, the desiccant element 14382 includes two opposing side portions 14385 that extend downwardly from a top portion 14383. The side portions 14385 may be configured to releasably engage the corresponding sides of the surgical staple cartridge 14200. In addition or in the alternative, the desiccant element 14382 may be removably coupled to the deck surface 14204 by an adhesive, for example. Alternatively, the desiccant element 14382 may comprise the top portion 14383 and not include the side portions 14385. In such instance, the desiccant element may be removably attached to the deck surface 14204 by an adhesive. In various arrangements, the adhesive may comprise a "double stick" or double-sided tape. However, other adhesive materials may be employed to removably or temporarily affix the desiccant element 14382 to the deck surface 14204. In another arrangement, the desiccant element 14382 may additionally comprise a plurality of staple retention protrusions (see FIG. 12) that protrude from the underside of the desiccant element 14382 and are configured to extend into the staple cavities 14220 as was discussed above. In one arrangement, the staple retention protrusions serve to non-movably retain the staples 14250 within their respective cavities 14220 as well as retain the desiccant element on the deck surface 14204 during storage and shipping. In other arrangements, however, an adhesive may be employed to removably adhere the desiccant element to the deck surface 14204, for example.

The desiccant element 14382 may comprise a 220 g/m² +/- 5% Invercote T2 paper per P-02-003 (Adaptiv) or other commercially available moisture absorbent board. The desiccant element 14382 may further comprise a blue desiccant material containing cobalt chloride which will change to a pink color when the desiccant material has reached its maximum adsorption capacity. This change in color would indicate a compromised package. In another arrangement, for example, the desiccant element 14382 may comprise a PH sensitive dye that changes color upon exposure to basic environments which may indicate corrosion of Mg to Mg(OH)2. Alternatively, the desiccant element 14382 may be treated with an irreversible hydrochromic dye such as, for example, patent blue V (tryphenylmethane) dye, cobalt II chloride or methyl violet, for example.

FIG. 20 illustrates another desiccant element 14384 that is formed to immovably support a surgical staple cartridge 14200 or a cartridge/retainer assembly 14330 during storage and shipping of a cartridge/retainer assembly 14330 within a hermetically-sealable container 14320'. The hermetically-sealable container 14320' may be fabricated from rigid plastic or other suitable material that may or may not be transparent. The hermetically-sealable container 14320 may be configured to support one or a plurality of cartridge/retainer assemblies 14330 and their respective desiccant elements 14384, for example. The hermetically-sealable container 14320' may include sides 14321 that form hermetic seals with each other. One side 14323 may be hinged or otherwise configured to facilitate opening of the hermetically-sealable container 14320'. In other arrangements, for example, the cartridge/retainer assembly 14330 and desiccant support element 14384 may be supported in a hermetically-sealable pouch 14322 or other hermetically-sealable container arrangement, for example.

As can be seen in FIG. 20, the desiccant element 14384 is configured to immovably support the cartridge/retainer assembly 14330 and serves to protect the cartridge/retainer assembly 14330 (or surgical staple cartridge 14200) and minimize movement thereof during shipping and storage within a hermetically-sealable container 14320', for example. In various instances, the desiccant element 14384 comprises a 220 g/m² +/- 5% Invercote T2 paper per P-02-003 (Adaptiv) or other commercially available moisture absorbent board that can be folded or otherwise fashioned into a supportive structure for the surgical staple cartridge 14200 or cartridge/retainer assembly 14330. The desiccant element 14384 may further comprise a blue desiccant material containing cobalt chloride which will change to a pink color when the desiccant material has reached its maximum adsorption capacity. This change in color would indicate a compromised package. In another arrangement, for example, the desiccant element 14382 may comprise a PH sensitive dye that changes color upon exposure to basic environments which may indicate corrosion of Mg to Mg(OH)2. Alterntively, the desiccant element 14384 may be treated with an irreversible hydrochromic dye such as, for example, patent blue V (tryphenylmethane) dye, cobalt II chloride or methyl violet, for example. Those of ordinary skill in the art will appreciate that the desiccant element 14384 may be fashioned into a variety of different supportive structures and configurations configured to non-movably support the surgical staple cartridge 14200 or cartridge/retainer assembly 14330, yet facilitate easy removal therefrom. For example, the desiccant element may comprise a moisture absorbent board that can be folded into a desired shape and retained in that shape by inserting tab portions into corresponding slots and/or using adhesive materials.

Turning next to FIG. 21, in one arrangement, the surgical staple cartridge 14200 comprises an RFID (radio frequency identification) chip or tag 14410 that is positioned in the cartridge body 14202. In the illustrated arrangement for example, the RFID chip 14410 is positioned in one of the lateral walls 14403 of the cartridge body 14202. A variety of RFID chips/tag configurations and systems are disclosed in further detail in U.S. Patent Application Publication No. 2020/0405296. Other RFID chips/tag configurations and systems are disclosed in U.S. Patent Application Publication 2021/0393268, entitled METHOD OF USING MULTIPLE RFID CHIPS WITH A SURGICAL ASSEMBLY.

The RFID chip 14410 is associated with and RFID system 14402 that comprises a scanner 14404 that is associated with the control system 14021. See FIG. 2. The RFID system 14402 can either be an active system, a passive system or comprise a combination of active and passive attributes. In passive systems, the onboard RFID chip does not comprise an internal power source and requires a reader or scanner to send a first signal, such as, for example an interrogation signal to the RFID chip. Active radio frequency identification systems also comprise an RFID chip and an RFID scanner. However, the RFID chip in an active RFID system comprises an internal power source. Active RFID systems utilize battery-powered RFID chips that are configured to continuously broadcast their own signal. One type of active RFID chip is commonly referred to as a "beacon." Such beacon RFID chips do not wait to receive a first signal from an RFID scanner. Instead, the beacon RFID chip continuously transmits its stored information. For example, the beacon can send out its information at an interval of every 3-5 seconds. Another type of active RFID chip comprises a transponder. In such systems, the RFID scanner transmits a signal first. The RFID transponder chip then sends a signal back to the RFID scanner with the relevant information. Such RFID transponder tag systems are efficient, as they conserve battery life when, for example, the RFID chip is out of range of the RFID scanner.

In the illustrated example, the RFID chip 14410 comprises or is associated with a sensor 14420 that is configured to track an environmental parameter within the container 14320. In one arrangement, for example, the sensor 14420 comprises a moisture sensor that is configured to monitor a moisture level within the container 14320. The RFID chip 14410 then transmits the moisture-level information to the scanner 14404 of the control system 14021. This transmission of information may occur when the surgical staple cartridge has been seated in the channel 14112 of the surgical stapling instrument 14000 or it may be transmitted to the control system 14021 prior to being seated in the channel 14112. To prevent the use of a surgical staple cartridge 14200 that has been exposed to a moisture level within the container 14320 that could have conceivably caused premature degrading of the bio-absorbable staples 14250 therein, in at least one arrangement, the control system 14021 is configured to prevent a staple firing stroke or other operation from being performed or permit the staple firing stroke or other operation to be performed based on feedback from the RFID system 14402. For example, the control system 14021 can execute the process 14023 illustrated in FIG. 22. Accordingly, the control circuit 14021 receives 14025 the detected moisture level information from the RFID chip 14410. The detected moisture level information M_{D} is then compared 14027 to a minimum acceptable level or compatible information M_{A} (which may be substantially zero or some other level determined to not cause premature degradation of the bio-absorbable staples 14250). If the detected moisture level information M_{D} is at or below the acceptable moisture level M_{A}, the control system 14021 permits 14029 operation of the surgical instrument systems (firing system, closure system). If the detected moisture level information M_{D} exceeds the acceptable moisture level M_{A}, the control system 14021 prevents 14031 operation of one or more of the surgical instrument systems.

In various instances, depending upon the particular composition of the staples 14250 stored in a particular surgical staple cartridge 14200, exposure of the staples 14250 to certain temperatures may, for example, result in age hardening of the staples 14250 or annealing of the staples 14250 and/or damage (melting of) to the staple coating. Thus, it is desirable to monitor the temperature level within the container 14320 while the surgical staple cartridge 14200 is being stored and shipped therein to ensure that the temperature level has not attained undesirable levels that could cause premature degradation of the staples 14250.

In another arrangement, for example, the sensor 14420 comprises a temperature sensor that is configured to monitor a temperature level within the container 14320. The RFID chip 14410 then transmits the temperature-level information T_{D} to the scanner 14404 of the control system 14021. This transmission of information may occur when the surgical staple cartridge 14200 has been seated in the cartridge-receiving channel 14112 of the surgical stapling instrument 14000 or it may be transmitted to the control system 14021 prior to being seated in the channel 14112. To prevent the use of a surgical staple cartridge 14200 that has been exposed to a temperature level within the container 14320 that could have conceivably caused premature degrading of the staples 14250 therein, in at least one arrangement, the control system 14021 is configured to prevent a staple firing stroke or other operation from being performed or permit the staple firing stroke or other operation to be performed based on feedback from the RFID system 14402. For example, the control system 14021 can execute the process 14033 illustrated in FIG. 23. Accordingly, the control system 14021 receives 14035 the detected temperature level information T_{D} from the RFID chip 14410. The detected temperature level information T_{D} is then compared 14037 to a minimum acceptable level or compatible information T_{A} (determined to not cause premature degradation of the staples 14250). If the detected temperature level information T_{D} is at or below the acceptable temperature level T_{A}, the control system 14021 permits 14039 operation of the surgical instrument systems (firing system, closure system, etc.).

In another arrangement, for example, the sensor 14420 is configured to monitor a temperature level as well as a moisture level within the container 14320. In other arrangements, a separate temperature sensor and a separate moisture sensor may be used. The separate temperature sensor and the separate moisture sensor may be associated with the RFID chip 14410 or the separate temperature sensor and the separate moisture sensor may each be associated with a dedicated RFID chip. In either case, the RFID chips would be configured to transmit the detected temperature level information T_{D} and detected moisture level information M_{D} to the scanner 14404/control system 14021. This transmission of information may occur when the surgical staple cartridge 14200 has been seated in the cartridge-receiving channel 14112 of the surgical stapling instrument 14000 or it may be transmitted to the control system 14021 prior to being seated in the channel 14112. To prevent the use of a surgical staple cartridge 14200 that has been exposed to a temperature level and/or a moisture level within the container 14320 that could have conceivably caused premature degrading of the staples 14250 therein, in at least one arrangement, the control system 14021 is configured to prevent a staple firing stroke or other operation from being performed or permit the staple firing stroke or other operation to be performed based on feedback from the RFID system 14402. For example, the control system 14021 can execute the process 14043 illustrated in FIG. 24. Accordingly, the control system 14021 receives 14045 the detected temperature level information T_{D} and the detected moisture level information M_{D} from the RFID chip(s) 14410. The detected temperature level information T_{D} is then compared 14047 to a minimum acceptable level or compatible information T_{A} (which may be determined to not cause premature degradation of the staples 14250). If the detected temperature level information T_{D} exceeds the acceptable temperature level T_{A}, the control system 14021 prevents 14041 operation of one or more of the surgical instrument systems. If the detected temperature level information T_{D} is at or below the acceptable temperature level T_{A}, the control system 14021 then compares 14051 the detected moisture level information M_{D} to a minimum acceptable level or compatible information M_{A} (determined to not cause premature degradation of the staples 14250). If the detected moisture level information M_{D} exceeds the acceptable moisture level M_{A}, the control system 14021 prevents 14049 operation of one or more of the surgical instrument systems. If the detected moisture level information M_{D} is at or below the acceptable moisture level M_{A}, the control system 14021 permits 14053 operation of the surgical instrument systems (firing system, closure system).

The staples 14250 may comprise a variety of different compositions and configurations disclosed herein. In certain instances, each composition of the staples 14250 may have unique sensitivities to a particular moisture level and/or temperature level. Thus, the staples 14250 in one surgical staple cartridge 14200 may be more sensitive to a particular moisture and/or temperature level than the staples 14250 in another surgical staple cartridge that may also be employed in the surgical stapling instrument 14000. In such instances, the surgical staple cartridge 14200 may comprise another RFID chip/tag (not shown) that contains identification information regarding the surgical staple cartridge 14200 including the particular composition of the staples 14250 stored therein. This information is then transmitted by the RFID chip/tag to the control system 14021. In other arrangements, such information could also be transmitted by RFID chip 14110. In either case, the control system 14021 then looks up/determines the acceptable temperature and/or moisture level information (compatible information) for the particular surgical cartridge 14200 that has been seated in the channel 14112 or is slated to be used with the surgical stapling instrument 14000. That compatible information for that particular surgical staple cartridge 14200 is used during the implementation of the processes described herein to determine whether the surgical staple cartridge 14200 was exposed to unacceptable temperature and/or moisture levels (for the bio-absorbable staples in that particular cartridge) within the container.

FIG. 25 illustrates a container 14320 that comprises an indicator member 14500 that has a sensor 14510 associated therewith. In one arrangement, the sensor 14510 comprises a moisture sensor that is configured to detect a moisture level within the container 14320 and provide a signal to the indicator member 14500 if the sensed moisture level exceeds a predetermined level which could cause, for example, premature degradation of the bio-absorbable staples 14250 that are stored in the surgical staple cartridge 14200. In one arrangement, the indicator member 14500 comprises a light emitting diode (LED). The indicator member 14500 and the sensor 14510 may each have an onboard power source such that when the moisture level inside of the container 14320 has exceeded the predetermined level, the LED is illuminated. The clinician would then be informed that the integrity of the staples 14250 may have been compromised due to exposure to moisture and then avoid use of the surgical staple cartridge 14200.

In an alternative arrangement, the sensor 14510 comprises a temperature sensor that is configured to detect a temperature level within the container 14320 and provide a signal to the indicator member 14500 if the sensed temperature level exceeds a predetermined level which could cause, for example, premature degradation of the staples 14250 that are stored in the surgical staple cartridge 14200. In one arrangement, the indicator member 14500 comprises a light emitting diode (LED). The indicator member 14500 and the sensor 14510 may each have an onboard power source such that when the temperature level inside of the container 14320 has exceeded the predetermined acceptable level, the LED 14500 is illuminated. The clinician would then be informed that the integrity of the staples 14250 may have been compromised due to an undesirable temperature within the container 14320 and then avoid use of the surgical staple cartridge.

In still another arrangement, the sensor 14510 (or separate sensors) is configured to sense a moisture level and a temperature level within the container 14320. If the moisture level exceeds the predetermined level or the temperature exceeds the predetermined level, an LED 14500 would be illuminated. Alternatively, after the control system 14021 compares the transmitted moisture level that was detected within the container 14320 and/or the transmitted temperature level that was detected within the container, the control system 14021 may activate another indicator that may be onboard the surgical stapling instrument 14000. For example, the indicator may comprise an LED, speaker, buzzer, haptic feedback system, notification screed onboard the surgical stapling instrument 14000 or otherwise associated with the surgical stapling instrument 14000, etc. to provide the clinician with an indication that the surgical staple cartridge 14200 may have been compromised and should not be used. In other instances, the control system may be configured to provide the clinician with an indication of the environmental history of a particular container and the cartridge/retainer assemblies or surgical staple cartridge retained therein. Such environmental history may be presented in table or graph form, for example, that is displayed on a display (not shown) associated with the surgical stapling instrument.

Turning to FIG. 26, in various examples, the container 14320 may be filled with a gas 14520 to provide the interior of the container 14320 with a desired environment. For example, the gas 14520 may comprise dry nitrogen or argon which serves to provide the interior of the container with an extremely low moisture and oxygen environment.

In various examples described herein, the container 14320 comprises a hermetically-sealable peel-pouch that is fabricated from an air/gas impermeable and moisture impermeable foil or plastic material. In other examples, the container 14320 is constructed of other suitable materials that can provide a hermetic seal. Other materials for use in constructing the container 14320 of packaging assembly 14300 will be apparent to those of ordinary skill in the art. The container 14320 may also comprise other hermetically-sealable structures that are fabricated from a gas and moisture impermeable material and are configured to store one or more surgical staple cartridges 14200 and/or one or more of the various cartridge/retainer assemblies 14330 disclosed herein. Such other container configurations may be fabricated from rigid material such as a plastic and may be formed with cartridge retention cavities or retention features for non-movably retaining one or more surgical staple cartridges 14200 and/or one or more cartridge/retainer assemblies 14330 therein while facilitating easy removal therefrom after the container has been opened. In various arrangements, a container may be fabricated from a transparent material or otherwise be configured to permit the contents of the container to be viewed without opening the container. In addition, or in the alternative, identifying indicia, such as, for example RFID tags, bar codes, QR codes, labels etc. may be applied to the outer surface of the container.

The various packaging assemblies, containers, staple retainers, desiccant elements disclosed herein offer solutions to many if not all of the challenges associated with storing and shipping surgical staple cartridges that contain bio-absorbable staples that are designed to degrade within a desired period of time after being formed in tissue. As was discussed above, the absorption of the staples may be driven by the exposure level to O₂, CO₂, and H₂O, which allows the staples to first form oxidation product and then be converted into salts and absorbed. If the staples are exposed to O₂, CO₂, and H₂O, for example, while being stored, shipped and otherwise prior to use, they may prematurely begin to degrade which could detrimentally affect their ability to properly form and retain tissue after deployment into the body for a desired period of time. The various arrangements disclosed herein serve to prevent the surgical staple cartridges containing such bio-absorbable staples from exposure to such elements prior to use while facilitating easy access thereto.

The various bio-absorbable staples disclosed herein may begin to prematurely deteriorate if exposed to moisture and/or higher temperatures. The various packaging assemblies and packaging systems disclosed herein may comprise sensors configured to monitor the moisture level and/or temperature level experienced by the surgical staple cartridge(s) or cartridge /retainer assemblies stored within the hermetically-sealable container. Such sensor arrangements may be associated with indicator members, LED's, etc. in the container and/or be associated with an RFID system in the surgical stapling instrument that is configured to prevent operation of the surgical stapling instrument when the moisture level and/or temperature level within the container has exceeded level(s) that might result in the premature degradation of the bio-absorbable staples. The various desiccant elements disclosed herein may also change color in the presence of moisture to provide the user with an indication that the staples in the surgical staple cartridge have been exposed to moisture and may have begun to prematurely degrade. In other instances, the staples may be treated with a vapor corrosion inhibitor and/or any one of the desiccant element configurations disclosed herein may be fabricated from a material configured to diffuse a vapor corrosion inhibitor therefrom in place of the desiccant material and in other instances in addition to the desiccant material.

Further to the above, some of the bio-absorbable staples disclosed herein are provided with a coating to slow oxidation process. Thus, various container, staple retainers, and desiccant elements disclosed herein serve to non-movably retain the surgical staple cartridges within their respective container and/or non-movably retain each staple within its respective staple cavity. In one arrangement for example, the staples are heated within their respective cavities to cause the coating on the portions of the staples that are in contact with the cavity walls and/or the staple driver to melt the coating to temporarily adhere the staples to the inner walls and/or the staple driver. Such arrangements prevent movement and/or migration of the individual staples and cartridges during storage and shipping which could otherwise damage the staple coating and cause the staples to prematurely degrade. It will also be appreciated that while the various packaging assemblies disclosed herein are particularly well suited for use with surgical staple cartridge that contain bio-absorbable staples, the person of ordinary skill in the art will appreciate that the various packaging assemblies disclosed herein may find equal utility when used in connection with surgical staple cartridges containing other forms of staples and/or fasteners.

As mentioned above, in many instances, after the staples have been installed in the surgical staple cartridge, the surgical staple cartridge is then sterilized utilizing, for example, gamma radiation, x-rays, high-energy electrons, beta radiation, ethylene oxide, plasma peroxide, steam etc. The various staple retainers, desiccant elements, adjuncts and cassettes disclosed herein may be coupled to a surgical staple cartridge prior to sterilization. In such instances, the staple retainers, desiccant elements, adjuncts and cassettes provide air/fluid paths for a sterilization medium to invade and be flushed from the interior portions of the cartridge base.

Various bio-absorbable staples disclosed herein may be coated with a coating to slow the oxidation of the staple core material. Thus, it may be advantageous to prevent or minimize damage to that coating as the staple is being formed through contain with a corresponding staple pocket in the anvil. Thus, any of the packaging assemblies and packaging systems disclosed herein may comprise or additionally comprise a pretreatment element that is configured to apply a pretreatment medium to the staple pockets prior to forming the staples. The pretreatment medium may comprise sodium sterates, silicones, absorbable polymers, and hydrogels. The pretreatment element may be associated with a staple retainer or be separate from the staple retainer and placed between the cartridge deck surface and the anvil after the staple retainer has been detached from the staple cartridge. The pretreatment element may be packaged in a dedicated package/pouch and included in the hermetically-sealable container with the surgical staple cartridge(s) or cartridge/retainer assemblies contained therein.

The various packaging assemblies and packaging systems disclosed herein may include a hermetically-sealable container and a staple retainer. The staple retainer may be configured to prevent movement of each staple within their individual cavity. The packaging assemblies and packaging systems may include a desiccant element contained within the container to absorb and reduce moisture within the container and/or the surgical staple cartridge(s). The desiccant element may be configured to be removably trapped between a staple retainer and a surgical staple cartridge and/or attached to the staple retainer or removably attached to the surgical staple cartridge and used without a staple retainer. The desiccant element may be configured to prevent movement of each staple within their individual cavity. The desiccant element may be configured to change color in the presence of moisture. Multiple desiccant elements may be employed within the container to also isolate the surgical staple cartridge or cartridge/reload assembly from vibration and shock. In other instances, the desiccant element may be configured to non-movably support a surgical staple cartridge or cartridge/retainer assembly therein. The container may be equipped with sensor(s) to detect the moisture level and/or temperature levels in the container and provide indications thereof. The detected levels may be used by the stapling instrument controller to prevent operation of the surgical stapling instrument if the levels exceed acceptable levels.

Different staple cartridges can have different types of staples stored therein. In some cases, a staple cartridge is sold and delivered to a clinician with one or more indicia thereon which indicate to the clinician the type of staples stored in the staple cartridge. The indicia may include words that inform the clinician that pure magnesium and magnesium alloy staples are contained therein. The indicia may include visual representations of the types of staples and their locations with the staple cartridge. For exmple, the indicia comprises a staple pattern than mimics the staples the pattern of staples stored in the enclosed staple cartridge. For instance, if the pure magnesium staples are stored in the proximal end and the distal end of the staple cartridge and the magnesium alloy staples are stored in between the proximal end and the distal end, solid black lines can be used as representations of the magnesium alloy staples while dashed black lines can be used as representations of the pure magnesium alloy staples. The dashed black lines, as opposed to the solid black lines, signify that the pure magnesium staples may bioabsorb faster than the magnesium alloy staples. Similarly, thick solid lines can be used to represent staples having a thicker wire diameter while thin solid lines can be used to represent staple staples having a thinner wire diameter, for example.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

## Claims

1. A packaging assembly (14300) comprising:
a surgical staple cartridge (14200) configured to be operably installed in a surgical stapling instrument (14000), wherein the surgical staple cartridge (14200) comprises a body (14202), a deck (14204), staple cavities (14220) defined in the deck (14204), and staples (14250) removably stored in the staple cavities (14220);
a container (14320) configured to store the surgical staple cartridge (14200) therein and permit the surgical staple cartridge (14200) to be removed therefrom; and
a desiccant element (14350, 14360, 14382);
**characterized in that:**
the staple cartridge further comprises a longitudinal slot (14206) defined in the deck (14204);
the desiccant element is removably attached to the deck (14204) of the surgical staple cartridge (14200) and covers the staple cavities (14220) in the cartridge deck (14204); and
the container (14320) is hermetically sealable with the cartridge (14200) and the desiccant element removably attached to the cartridge deck (14204) stored therein.

2. The packaging assembly (14300) of claim 1, further comprising a staple retainer (14310) received on the deck (14204) of the surgical staple cartridge (14200) and sized to cover the staple cavities (14220) in the cartridge deck (14204), wherein said staple retainer (14310) is removably coupled to the body (14202), wherein said desiccant element is removably positioned between the staple retainer (14310) and the deck (14204) of the surgical staple cartridge (14200).

3. The packaging assembly (14300) of Claim 2, wherein said desiccant element protrudes into each staple cavity (14220) in the deck (14204).

4. The packaging assembly (14300) of Claim 3, wherein said desiccant element (14360) comprises:
an elongate body portion;
a plurality of retention protrusions (14364) protruding from said elongate body portion, wherein each said retention protrusion (14364) corresponds to one of the staple cavities (14220) and protrudes therein.

5. The packaging assembly (14300) of Claim 4, wherein each said retention protrusion (14364) retainingly engages at least one staple (14250) stored in the corresponding staple cavity (14220).

6. The packaging assembly (14300) of Claim 2, wherein said staple retainer (14310) defines a deck-facing surface (14313) confronting the deck (14204), and wherein said desiccant element (14350) is attached to said deck-facing surface (14313).

7. The packaging assembly (14300) of Claim 2, wherein the cartridge body (14202) comprises a first cartridge body side (14203) and a second cartridge body side (14205), wherein the first cartridge body side (14203) and second cartridge body side (14205) depend from the deck (14204), wherein the cartridge body (14202) further comprises a bottom surface (14207) extending between the first cartridge body side (14203) and the second cartridge body side (14205), and wherein said desiccant element (14390) comprises:
a desiccant element top portion (14392) received on the deck (14204) and covering all of the staple cavities (14220) therein;
a first desiccant element side (14393), wherein the first desiccant element side (14393) depends from said desiccant element top portion (14392) and corresponds to the first cartridge body side (14203); and
a second desiccant element side (14395), wherein said second desiccant element side (14395) depends from said desiccant top portion (14392) and corresponds to the second cartridge body side (14205).

8. The packaging assembly (14300) of Claim 7, wherein said desiccant element (14390) further comprises a desiccant element bottom portion (14394) attached to said first desiccant element side (14393) and said second desiccant element side (14395) and extends therebetween, and wherein said desiccant element bottom portion (14394) is adjacent at least a portion of the bottom surface (14207) of the cartridge body (14200).

9. The packaging assembly (14300) of Claim 2 or Claim 7, wherein said desiccant element is configured to change color in the presence of moisture.

10. The packaging assembly (14300) of Claim 2, wherein the staples (14250) comprise bio-absorbable staples comprised of an alloy configured to accelerate corrosion of the bio-absorbable staples.

11. The packaging assembly (14300) of Claim 2, wherein said hermetically-sealable container (14320) is filled with Nitrogen gas or Argon gas.

12. The packaging assembly (14300) of Claim 2, further comprising a moisture or temperature sensor (14420, 14510) in said hermetically-sealable container (14320) configured to assess a moisture or temperature level within said hermetically-sealable container (14320) and provide an indication thereof.

13. The packaging assembly (14300) of Claim 12, wherein said moisture or temperature sensor (14420, 14510) is configured to provide said indication of said moisture or temperature level within said hermetically-sealable container (14320) only when said moisture or temperature level exceeds a predetermined magnitude.

14. The packaging assembly (14300) of Claim 13, further comprising an RFID chip (14410) on the body (14202), wherein said moisture or temperature sensor (14420, 14510) communicates moisture or temperature level information concerning said moisture or temperature level within said hermetically-sealable container (14320) to said RFID chip (14410), wherein a controller (14021) of the surgical stapling instrument (14000) comprises an RFID scanner (14404) configured to receive said moisture or temperature level information from said RFID chip (14410), wherein the controller (14021) is configured to compare said moisture or temperature level information transmitted by said RFID chip (14410) to a set of compatible information, and wherein the controller (14021) is configured to prevent the surgical stapling instrument (14000) from performing at least one function if said moisture or temperature level information is not found in the set of compatible information.

15. The packaging assembly (14300) of Claim 13, further comprising a light emitting diode (14500) in said hermetically-sealable container (14320), wherein said light emitting diode (14500) communicates with said moisture or temperature sensor (14420, 14510), and wherein said light emitting diode (14500) is illuminated when said moisture or temperature level exceeds said predetermined magnitude.

## Patentansprüche

1. Verpackungsanordnung (14300), umfassend:
ein chirurgisches Klammermagazin (14200), das konfiguriert ist, um betriebsbereit in einem chirurgischen Klammerinstrument (14000) installiert zu werden, wobei das chirurgische Klammermagazin (14200) einen Körper (14202), ein Deck (14204), in dem Deck (14204) definierte Klammerhohlräume (14220) und entnehmbar in den Klammerhohlräumen (14220) gelagerte Klammern (14250) umfasst;
einen Behälter (14320), der konfiguriert ist, um das chirurgische Klammermagazin (14200) darin aufzubewahren und die Entnahme des chirurgischen Klammermagazins (14200) aus dem Behälter zu ermöglichen; und
ein Trockenmittelelement (14350, 14360, 14382);
**dadurch gekennzeichnet, dass:**
das Klammermagazin ferner einen Längsschlitz (14206) umfasst, der im Deck (14204) definiert ist;
das Trockenmittelelement abnehmbar an dem Deck (14204) des chirurgischen Klammermagazins (14200) angebracht ist und die Klammerhohlräume (14220) im Magazindeck (14204) abdeckt; und
der Behälter (14320) hermetisch verschließbar ist, wobei das Magazin (14200) und das Trockenmittelelement abnehmbar an dem darin aufbewahrten Magazindeck (14204) befestigt sind.

2. Verpackungsanordnung (14300) nach Anspruch 1, ferner umfassend einen Klammerhalter (14310), der auf dem Deck (14204) des chirurgischen Klammermagazins (14200) aufgenommen und so bemessen ist, dass er die Klammerhohlräume (14220) im Magazindeck (14204) abdeckt, wobei der Klammerhalter (14310) abnehmbar mit dem Körper (14202) verbunden ist, wobei das Trockenmittelelement abnehmbar zwischen dem Klammerhalter (14310) und dem Deck (14204) des chirurgischen Klammermagazins (14200) positioniert ist.

3. Verpackungsanordnung (14300) nach Anspruch 2, wobei das Trockenmittelelement in jeden Klammerhohlraum (14220) im Deck (14204) hineinragt.

4. Verpackungsanordnung (14300) nach Anspruch 3, wobei das Trockenmittelelement (14360) umfasst:
einen länglichen Körperabschnitt;
eine Vielzahl von Haltevorsprüngen (14364), die aus dem länglichen Körperabschnitt hervorstehen, wobei jeder Haltevorsprung (14364) einem der Klammerhohlräume (14220) entspricht und in diesen hineinragt.

5. Verpackungsanordnung (14300) nach Anspruch 4, wobei jeder Haltevorsprung (14364) mindestens eine Klammer (14250) festhaltend eingreift, die in dem entsprechenden Klammerhohlraum (14220) aufbewahrt wird.

6. Verpackungsanordnung (14300) nach Anspruch 2, wobei der Klammerhalter (14310) eine dem Deck (14204) zugewandte Oberfläche (14313) bildet, und wobei das Trockenmittelelement (14350) an der dem Deck zugewandten Oberfläche (14313) angebracht ist.

7. Verpackungsanordnung (14300) nach Anspruch 2, wobei der Magazinkörper (14202) eine erste Magazinkörperseite (14203) und eine zweite Magazinkörperseite (14205) umfasst, wobei die erste Magazinkörperseite (14203) und die zweite Magazinkörperseite (14205) von dem Deck (14204) abhängen, wobei der Magazinkörper (14202) ferner eine Bodenoberfläche (14207) umfasst, die sich zwischen der ersten Magazinkörperseite (14203) und der zweiten Magazinkörperseite (14205) erstreckt, und wobei das Trockenmittelelement (14390) umfasst:
einen oberen Abschnitt (14392) des Trockenmittelelements, der auf dem Deck (14204) aufgenommen ist und alle Klammerhohlräume (14220) darin abdeckt;
eine erste Trockenmittelelementseite (14393), wobei die erste Trockenmittelelementseite (14393) von dem oberen Abschnitt (14392) des Trockenmittelelements abhängt und der ersten Magazinkörperseite (14203) entspricht; und
eine zweite Trockenmittelelementseite (14395), wobei die zweite Trockenmittelelementseite (14395) von dem oberen Trockenmittelabschnitt (14392) abhängt und der zweiten Magazinkörperseite (14205) entspricht.

8. Verpackungsanordnung (14300) nach Anspruch 7, wobei das Trockenmittelelement (14390) ferner einen Trockenmittelelement-Bodenabschnitt (14394) umfasst, der an der ersten Trockenmittelelementseite (14393) und der zweiten Trockenmittelelementseite (14395) angebracht ist und sich dazwischen erstreckt, und wobei der Trockenmittelelement-Bodenabschnitt (14394) an mindestens einen Abschnitt der Bodenoberfläche (14207) des Magazinkörpers (14200) angrenzt.

9. Verpackungsanordnung (14300) nach Anspruch 2 oder Anspruch 7, wobei das Trockenmittelelement konfiguriert ist, um bei Vorhandensein von Feuchtigkeit seine Farbe ändert.

10. Verpackungsanordnung (14300) nach Anspruch 2, wobei die Klammern (14250) biologisch absorbierbare Klammern umfassen, die aus einer Legierung bestehen, die konfiguriert ist, um die Korrosion der biologisch absorbierbaren Klammern zu beschleunigen.

11. Verpackungsanordnung (14300) nach Anspruch 2, wobei der hermetisch verschließbare Behälter (14320) mit Stickstoffgas oder Argongas gefüllt ist.

12. Verpackungsanordnung (14300) nach Anspruch 2, ferner umfassend einen Feuchtigkeits- oder Temperatursensor (14420, 14510) in dem hermetisch verschließbaren Behälter (14320), der so konfiguriert ist, dass er ein Feuchtigkeits- oder Temperaturniveau innerhalb des hermetisch verschließbaren Behälters (14320) ermittelt und eine Anzeige darüber bereitstellt.

13. Verpackungsanordnung (14300) nach Anspruch 12, wobei der Feuchtigkeits- oder Temperatursensor (14420, 14510) so konfiguriert ist, dass er die Anzeige des Feuchtigkeits- oder Temperaturniveaus innerhalb des hermetisch verschließbaren Behälters (14320) nur dann bereitstellt, wenn das Feuchtigkeits- oder Temperaturniveau einen vorgegebenen Wert überschreitet.

14. Verpackungsanordnung (14300) nach Anspruch 13, ferner umfassend einen RFID-Chip (14410) auf dem Körper (14202), wobei der Feuchtigkeits- oder Temperatursensor (14420, 14510) Feuchtigkeits- oder Temperaturniveauinformationen bezüglich des Feuchtigkeits- oder Temperaturniveaus innerhalb des hermetisch verschließbaren Behälters (14320) an den RFID-Chip (14410) übermittelt, wobei eine Steuerung (14021) des chirurgischen Klammerinstruments (14000) einen RFID-Scanner (14404) umfasst, der dazu konfiguriert ist, die Feuchtigkeits- oder Temperaturniveauinformationen von dem RFID-Chip (14410) zu empfangen, wobei die Steuerung (14021) dazu konfiguriert ist, die von dem RFID-Chip (14410) übertragenen Feuchtigkeits- oder Temperaturniveauinformationen mit einem Satz kompatibler Informationen zu vergleichen, und wobei die Steuerung (14021) dazu konfiguriert ist, das chirurgische Klammerinstrument (14000) daran zu hindern, mindestens eine Funktion auszuführen, wenn die Feuchtigkeits- oder Temperaturniveauinformationen nicht in dem Satz kompatibler Informationen gefunden werden.

15. Verpackungsanordnung (14300) nach Anspruch 13, ferner umfassend eine Leuchtdiode (14500) in dem hermetisch verschließbaren Behälter (14320) umfasst, wobei die Leuchtdiode (14500) mit dem Feuchtigkeits- oder Temperatursensor (14420, 14510) kommuniziert, und wobei die Leuchtdiode (14500) aufleuchtet, wenn das Feuchtigkeits- oder Temperaturniveau die vorgegebene Größenordnung überschreitet.

## Revendications

1. Ensemble conditionnement (14300) comprenant :
une cartouche d'agrafes chirurgicales (14200) conçue pour être installée fonctionnellement dans un instrument d'agrafage chirurgical (14000), dans lequel la cartouche d'agrafes chirurgicales (14200) comprend un corps (14202), une platine (14204), des cavités d'agrafe (14220) définies dans la platine (14204), et des agrafes (14250) stockées de manière amovible dans les cavités d'agrafe (14220) ;
un conteneur (14320) conçu pour stocker la cartouche d'agrafes chirurgicales (14200) à l'intérieur de celui-ci et permettre à la cartouche d'agrafes chirurgicales (14200) d'être retirée de celui-ci ; et
un élément siccatif (14350, 14360, 14382) ;
**caractérisé en ce que** :
la cartouche d'agrafes comprend en outre une encoche longitudinale (14206) définie dans la platine (14204) ;
l'élément siccatif est attaché de manière amovible à la platine (14204) de la cartouche d'agrafes chirurgicales (14200) et couvre les cavités d'agrafe (14220) dans la platine (14204) de cartouche ; et
le conteneur (14320) peut être scellé hermétiquement avec la cartouche (14200) et l'élément siccatif attaché de façon amovible à la platine (14204) de cartouche stocké à l'intérieur de celui-ci.

2. Ensemble conditionnement (14300) selon la revendication 1, comprenant en outre un élément de retenue d'agrafe (14310) reçu sur la platine (14204) de la cartouche d'agrafes chirurgicales (14200) et dimensionné pour couvrir les cavités d'agrafe (14220) dans la platine (14204) de cartouche, dans lequel ledit élément de retenue d'agrafe (14310) est accouplé de manière amovible au corps (14202), dans lequel ledit élément siccatif est positionné de manière amovible entre l'élément de retenue d'agrafe (14310) et la platine (14204) de la cartouche d'agrafes chirurgicales (14200).

3. Ensemble conditionnement (14300) selon la revendication 2, dans lequel ledit élément siccatif fait saillie dans chaque cavité d'agrafe (14220) dans la platine (14204).

4. Ensemble conditionnement (14300) selon la revendication 3, dans lequel ledit élément siccatif (14360) comprend :
une partie de corps allongée ;
une pluralité de saillies de rétention (14364) faisant saillie de ladite partie de corps allongée, dans lequel chaque saillie de rétention (14364) précitée correspond à l'une des cavités d'agrafe (14220) et fait saillie à l'intérieur de celle-ci.

5. Ensemble conditionnement (14300) selon la revendication 4, dans lequel chaque saillie de rétention (14364) précitée vient en prise de manière retenue avec au moins une agrafe (14250) stockée dans la cavité d'agrafe (14220) correspondante.

6. Ensemble conditionnement (14300) selon la revendication 2, dans lequel ledit élément de retenue d'agrafe (14310) définit une surface orientée vers la platine (14313) faisant face vers la platine (14204), et dans lequel ledit élément siccatif (14350) est attaché à ladite surface orientée vers la platine (14313).

7. Ensemble conditionnement (14300) selon la revendication 2, dans lequel le corps de cartouche (14202) comprend un premier côté de corps de cartouche (14203) et un second côté de corps de cartouche (14205), dans lequel le premier côté de corps de cartouche (14203) et le second côté de corps de cartouche (14205) dépendent de la platine (14204), dans lequel le corps de cartouche (14202) comprend en outre une surface inférieure (14207) s'étendant entre le premier côté de corps de cartouche (14203) et le second côté de corps de cartouche (14205), et dans lequel ledit élément siccatif (14390) comprend :
une partie supérieure d'élément siccatif (14392) reçue sur la platine (14204) et couvrant la totalité des cavités d'agrafe (14220) à l'intérieur de celle-ci ;
un premier côté d'élément siccatif (14393), dans lequel le premier côté d'élément siccatif (14393) dépend de ladite partie supérieure d'élément siccatif (14392) et correspond au premier côté de corps de cartouche (14203) ; et
un second côté d'élément siccatif (14395), dans lequel ledit second côté d'élément siccatif (14395) dépend de ladite partie supérieure de siccatif (14392) et correspond au second côté de corps de cartouche (14205).

8. Ensemble conditionnement (14300) selon la revendication 7, dans lequel ledit élément siccatif (14390) comprend en outre une partie inférieure d'élément siccatif (14394) attachée audit premier côté d'élément siccatif (14393) et audit second côté d'élément siccatif (14395) et s'étend entre eux, et dans lequel ladite partie inférieure d'élément siccatif (14394) est adjacente à au moins une partie de la surface inférieure (14207) du corps de cartouche (14200).

9. Ensemble conditionnement (14300) selon la revendication 2 ou la revendication 7, dans lequel ledit élément siccatif est conçu pour changer de couleur en présence d'humidité.

10. Ensemble conditionnement (14300) selon la revendication 2, dans lequel les agrafes (14250) comprennent des agrafes biorésorbables constituées d'un alliage conçu pour accélérer la corrosion des agrafes biorésorbables.

11. Ensemble conditionnement (14300) selon la revendication 2, dans lequel ledit conteneur (14320) scellable hermétiquement est rempli d'azote gazeux ou d'argon gazeux.

12. Ensemble conditionnement (14300) selon la revendication 2, comprenant en outre un capteur d'humidité ou de température (14420, 14510) dans ledit conteneur (14320) scellable hermétiquement conçu pour évaluer un niveau d'humidité ou de température au sein dudit conteneur (14320) scellable hermétiquement et fournir une indication de celui-ci.

13. Ensemble conditionnement (14300) selon la revendication 12, dans lequel ledit capteur d'humidité ou de température (14420, 14510) est configuré pour fournir ladite indication dudit niveau d'humidité ou de température au sein dudit conteneur (14320) scellable hermétiquement uniquement lorsque ledit niveau d'humidité ou de température dépasse un ordre de grandeur prédéterminé.

14. Ensemble conditionnement (14300) selon la revendication 13, comprenant en outre une puce RFID (14410) sur le corps (14202), dans lequel ledit capteur d'humidité ou de température (14420, 14510) communique des informations de niveau d'humidité ou de température concernant ledit niveau d'humidité ou de température au sein dudit conteneur (14320) scellable hermétiquement à ladite puce RFID (14410), dans lequel un organe de commande (14021) de l'instrument d'agrafage chirurgical (14000) comprend un scanneur RFID (14404) configuré pour recevoir lesdites informations de niveau d'humidité ou de température en provenance de ladite puce RFID (14410), dans lequel l'organe de commande (14021) est configuré pour comparer lesdites informations de niveau d'humidité ou de température transmises par ladite puce RFID (14410) à un ensemble d'informations compatibles, et dans lequel l'organe de commande (14021) est configuré pour empêcher l'instrument d'agrafage chirurgical (14000) de mettre en œuvre au moins une fonction si lesdites informations de niveau d'humidité ou de température ne sont pas trouvées dans l'ensemble d'informations compatibles.

15. Ensemble conditionnement (14300) selon la revendication 13, comprenant en outre une diode électroluminescente (14500) dans ledit conteneur (14320) scellable hermétiquement, dans lequel ladite diode électroluminescente (14500) communique avec ledit capteur d'humidité ou de température (14420, 14510), et dans lequel ladite diode électroluminescente (14500) est éclairée lorsque ledit niveau d'humidité ou de température dépasse ledit ordre de grandeur prédéterminé.
